# EUROPEAN PATENT SPECIFICATION

(11) **EP 0 230 052 B1**
(45) Date of publication and mention of the grant of the patent: **06.04.1994**
(21) Application number: 86118104.8
(22) Date of filing: 29.12.1986
(51) Int. Cl.: C07K 7/00, C07K 5/00, C07K 1/14, A61K 37/02, C12Q 1/04, A61K 45/05, A61K 35/14

(54) **Immunoamplifiers and related Compositions**
Immunverstärker und verwandte Zusammensetzungen
Immuno-amplificateurs et compositions apparentées

(30) Priority: 26.12.1985 US 813632; 02.09.1986 US 902683
(43) Date of publication of application: 29.07.1987
(73) Proprietor: Gottlieb, Arthur A., New Orleans Louisiana 70115 (US)
(72) Inventor: Gottlieb, Arthur A., New Orleans Louisiana 70115 (US)
(74) Representative: Popp, Eugen, Dr.

(56) References cited:
- EP-A- 0 042 064
- EP-A- 0 109 089
- EP-A- 0 173 889
- WO-A-81/03279
- US-A- 4 468 379
- CHEMICAL ABSTRACTS, vol. 100, no. 9, 27 February 1984, Columbus, OH (US); A.A. GOTTLIEB et al., p. 454, no. 66411z#
- CHEMICAL ABSTRACTS, vol. 105, no. 7, 18 August 1986, Columbus, OH (US); A.A. GOTTLIEB et al., p. 494, no. 59226t#
- CHEMICAL ABSTRACTS, vol. 99, no. 1, 04 July 1983, Columbus, OH (US); p. 272, no. 2724g#
- CHEMICAL ABSTRACTS, vol. 99, no. 17, 24 October 1983, Columbus, OPH (US); S. MOUSA et al., pp. 71-72, no. 133892h#

## Description

This application is based on U.S. Pat. App. Ser. Nos. 813,632 ("Tripeptides affecting immune response"), filed December 26, 1985, and 902,683 ("Immunoamplifiers and related compositions"), filed September 2, 1986. Convention priority is claimed as to such dates with regard to the subject matter hereof, which is described in such applications.

### BACKGROUND OF THE INVENTION

This invention concerns cell-mediated immunity and pathological conditions associated with a deficiency in cell-mediated immunity. Such conditions include the Acquired Immune Deficiency Syndrome (AIDS), caused by the Human Immunodeficiency Virus, and AIDS-Related Complex (ARC).

A typical manifestation of cell-mediated immunity is the delayed hypersensitivity (DH) skin reaction. A DH skin reaction occurs when an appropriate antigen is injected subcutaneously. Within 24 to 48 hours, local inflammation (erythema) and a swelling and thickening (induration) are observed in a sensitive individual. The DH reaction typically involves perivascular infiltration of lymphocytes and monocytes in the inflamed area. The cells seen at the site of a DH reaction are derived from the peripheral blood leukocyte (PBL) population.

Other reactions of the cells which mediate the immune response to a challenge from an antigen include the following, among others: proliferation of cells bearing specific sensitivity to a given antigen; induction and multiplication of cells mediating a variety of immune functions, including antibody production; and reactions against foreign cells and tumors.

The present invention relates to the discovery of (1) processes for extracting "amplifiers" of the immunity system, which are isolated from dialyzed extracts of leukocytes, and (2) the amplifiers themselves that are so extracted (claimed herein together with pharmaceutical compositions containing them and methods of using them). These amplifiers profoundly affect the quality and quantity of cell-mediated immunity responses; are useful in the treatment of a variety of clinical conditions characterized by inadequate reaction to antigens; are useful in the alleviation of certain anergic conditions; and are useful in the treatment of certain autoimmune conditions.

Background relevant to the instant application is found in the present inventor's U.S. Patent No. 4,468,379, issued August 28, 1984 (hereinafter referred to as the '379 patent). Other useful background is found in the inventor's EPO application 85110104.8, published with EPO pub. no. 0173889 (12.03.86).

The prior art in this field is discussed in the '379 patent. That discussion is incorporated herein by reference.

Much of the prior art concerns the so-called "transfer factor," a product very different from the subject matter of this invention and in some ways antithetical in concept thereto. The concept of "transfer factor" is as follows: A "transfer factor" preparation is made from leukocytes of a donor known to be sensitive to a given antigen. The preparation is injected subcutaneously into the skin of a recipient known to be insensitive to the given antigen. Subsequently the recipient is challenged with the given antigen. A a DH response is then observed. Normally, the recipient, in the absence of the injected "transfer factor" preparation, would have been unresponsive to the challenge from the given antigen.

Progress in fractionating and characterizing "transfer factor" preparations has been impeded by an almost total lack of associated structural or chemical criteria. It is unclear whether the term "transfer factor" is used to refer to a single biochemical entity or rather to a mixture of materials of various kinds. The inventor believes that such preparations typically contain various different molecules and entities.

**Amplifiers.** An amplifier (also termed at times "immunoamplifier") is any substance or material that amplifies the onset, or rate or intensity, of the immune response of a human or animal to the reintroduction of an antigen to which the human or animal has previously been exposed. Hence, "amplifier" activity may be considered that specific immune system activity characterized, in general terms, by the production of a greater than normal immune response (faster or stronger, or both) in a recipient, following injection of an antigen to which the recipient is already sensitive.

The term "amplifier" is used, at times herein, as a noun or an adjective, to describe biologic agents or materials. Such material may also contain extraneous material without amplifier activity. The term "material" is used herein to describe compositions of matter that are or may be mixtures of different molecular species.

Amplifier material is useful in the treatment of anergic conditions and conditions of immune deficiency, both local and systemic, as AIDS and related conditions. The inventor has also discovered that amplifier material is useful in the treatment of autoimmune disorders, as disclosed in pending U.S. Pat. App. Ser. No. 848,210 ("Treatment of autoimmune disorders with immunoamplifiers"), filed April 4, 1986.

Amplifiers are not transfer factors, and transfer factors are not amplifiers, as defined above. First, the effect of a transfer factor is observed in a recipient who has not previously been exposed to a given antigen, while the effect of an amplifier is observed only upon or following reintroduction of an antigen to which the recipient was previously (or is concurrently) exposed. Furthermore, transfer factor effects are specific with respect to a given antigen, but amplifiers exert non-specific effects with respect to any antigen to which the recipient was previously exposed.

In the '379 patent, processes for the extraction of, among other things, six amplifiers (designated amplifiers 1-6) were described. The processes were based on high-pressure reverse-phase liquid chromatography (HPLC), using an ethanol-in-water gradient and octadecylsilane as the basic chromatography system. Various amplifiers elute at specified parts of the ethanol-in-water gradient, and can be identified in terms of the ethanol concentration of the material ("effluent") coming off the chromatography column (a physical or chemical property termed "O.S. elutability").

### SUMMARY OF THE INVENTION

The inventor has discovered additional processes for the extraction of amplifiers, and has discovered amplifier materials that can be extracted from leukocyte preparations by such processes. One chromatography system of the instant invention uses acetonitrile-phosphate aqueous gradients, and permits the isolation of approximately four to seven amplifier materials. Other chromatography systems are also disclosed hereinafter.

A significant advantage of the newly discovered processes is the elimination of the need for some of the steps of the original process and the easier and perhaps more effective extraction processes now provided, which allow a much more economical manufacture of amplifiers and much greater volume of production. Also, the apparent greater purity of the products of the newer processes disclosed herein and the further elimination of extraneous material from them gives them an important advantage, since the products of these processes are intended for medical use. Uses and compositions for using the foregoing amplifier materials are also disclosed.

### DESCRIPTION OF THE DRAWINGS

Figure 1 is a graph of 210 nm ultraviolet absorption of the effluent as it elutes from the HPLC column in the pH 5 extraction process of Example 4. The vertical axis represents absorbance units (full scale = 0.32 units), and the horizontal axis represents time (2 minutes/division). The fractions of effluent material are identified by name (Greek letters such as Beta).

Figures 1A through 1C are similar graphs, where the flow rate in the HPLC column is varied.

Figure 2 is a similar graph for the purification process of Example 5, on Material Beta. Figures 2A and 2B are similar graphs with respect to Material Zeta and Material Eta, respectively.

Figure 3 is a similar graph for the pH 2.5 extraction process of Example 6.

### DETAILED DESCRIPTION OF THE PREFERRED EMBODIMENT

In the following discussion, procedures are described in which materials were obtained from human donors and test measurements were made on human recipients. The procedures and reagents were chosen to provide sterile and non-toxic products for human treatment. Despite the toxicity of the solvent acetonitrile (CH₃CN), which is used in the extraction process for these materials, the end products of the processes are free of acetonitrile and they are nontoxic.

### I. Extraction of Small M.W. Fractions

The initial step in the preparation of the modulator material of this invention is preparation of leukocyte pellets, followed by separation of the small M.W. (under 3500) fractions of leukocyte extract of interest herein.

### EXAMPLE 1 -- Preparation and Filtration of Leukocyte Extracts

Leukocyte pellets were prepared by the methods of Example 1 of the '379 patent. "S" (or "small" M.W.) fraction of the extract is isolated by dialysis in accordance with Example 2 of the '379 patent or by an equivalent process such as ultrafiltration.

The next step in the preparation is gel filtration of the S fraction. This partially purifies the material of interest from other material, and separates the S-fraction material into a number of separate fractions having different characteristics. The following two procedures of the '379 patent (Examples 2 and 3) have been found unnecessary to carry out the pH 5 process of Example 4 of this application. It has not been verified, however, that they can be omitted with impunity in the pH 2.5 process of Example 6 of this application.

### EXAMPLE 2 -- Gel Filtration

Gel filtration was carried out on the leukocyte extract of preceding Example 1, in accordance with the method of Example 4 of the '379 patent. The resulting fractions were collected and set aside. (Note: Gel exclusion chromatography is described in the '379 patent as an equivalent of gel filtration.)

### EXAMPLE 3 -- Assay of Fractions

The fractions of preceding Example 2 are assayed in accordance with the procedure of the '379 patent. The major and secondary fluorescamine-reactive ("fluram-reactive") peaks were detected and those fractions were selected and set aside for further purification, described below.

### II. Reverse Phase Liquid Chromatography

Further purification of the material of preceding Examples 1 to 3 was carried out by reverse-phase high pressure liquid chromatography ("HPLC") processes discovered by the inventor.

The first process (hereinafter referred to as the pH 5 process) may be used with the material of any of preceding Examples 1, 2, or 3. The second process (hereinafter referred to as the pH 2.5 process) may be used with the material of preceding Example 3, and it is not known whether effective results can be obtained by starting with the material of Example 1 or 2. Each process uses an octadecylsilane ("O.S.") resin column eluted with a gradient of acetonitrile in aqueous phosphate solution. (All percentage references to gradients hereinafter are on a v/v basis.) The acetonitrile is HPLC grade (J.T. Baker Co., Phillipsburg, N.J.). The O.S. is a Waters Associates (Milford, Mass.) "mu Bondapak"™ C18 Octadecyl Silane reverse-phase analytical column (0.39 cm (ID) x 30 cm). The apparatus is a Series 3B Perkin-Elmer dual-pump, high-pressure, liquid chromatograph. The Perkin-Elmer machine includes an ultraviolet absorbance detector, which scans the column output at absorption 210 nm.

### A. Measurement of gradient portions

**Visual display.** The Perkin-Elmer Series 3B High Pressure Liquid Chromatograph can be programmed to operate at a specified flow rate, such as 1.0 ml/min, the rate that was used herein. Samples can then be collected at 1.0 minute intervals. A 0% to 20% solvent gradient can be programmed to run, for example, from 0% to 20% in 20 minutes. This divides the gradient into 20 1-minute intervals, during each of which intervals a 1.0 ml sample may be collected. Each such 1.0 ml sample should, in principle, represent a 1% change in solvent concentration. The visual display of the Perkin-Elmer machine shows the solvent concentration going into the column in accordance with the foregoing data.

The actual solvent concentration in the effluent from the column may be measured, for example, by refractive indices of effluent calibrated against a standard curve. As explained in the '379 patent, in the earlier portions of the gradient the solvent concentration of the effluent is substantially less than the concentration of solvent being programmed (at that moment) into the column of the machine.

It is possible to calibrate these columns, and important to do so in order to facilitate repeatable results, because the solvent concentration coming out of the column (rather than going in) is the important factor. This calibration can be done, with quite some difficulty in the case of some solvents (such as acetonitrile), by measurement of the effluent's refractive index and calculating actual solvent concentration therefrom. It is also possible to estimate or calculate output solvent concentration from input solvent concentration data by various other means such as use of radioactive or fluorescent tracers, and in that manner develop a calibration curve. It is believed better, if possible, to use some absolute measure of concentration of effluent solvent, rather than elution time or tube number, as a measure of actual effluent solvent concentration. It should be noted, however, that for any gradient system, where all other parameters remain constant, it is possible to use elution time as a measure or index, because there will be a regular correlation.

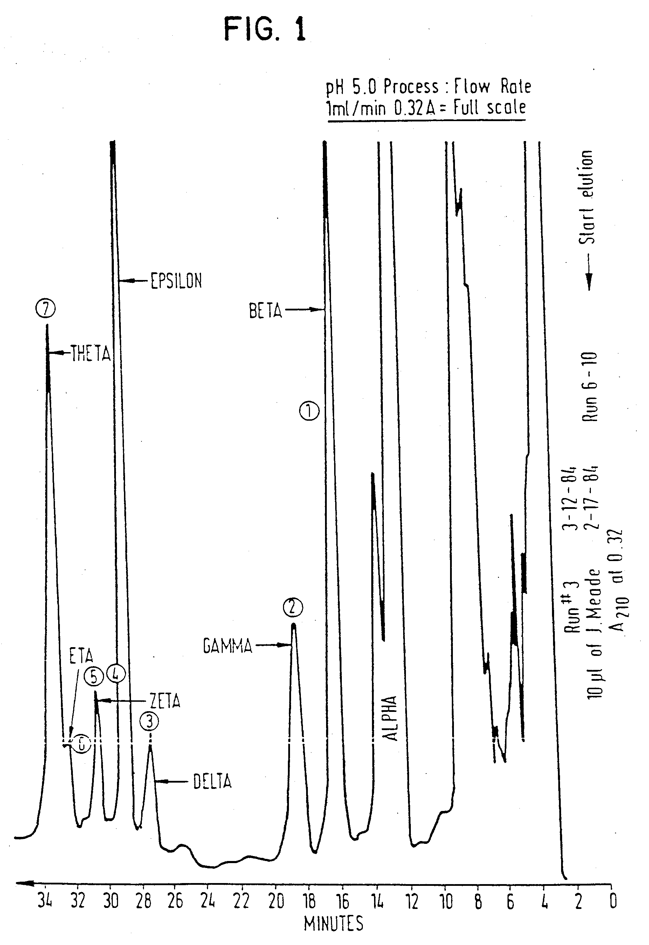

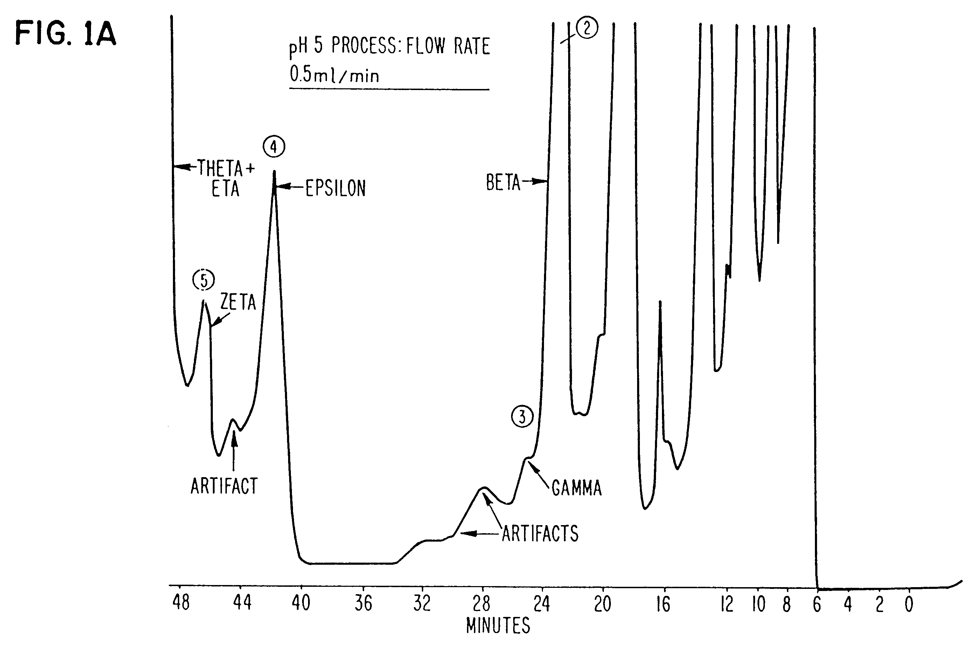

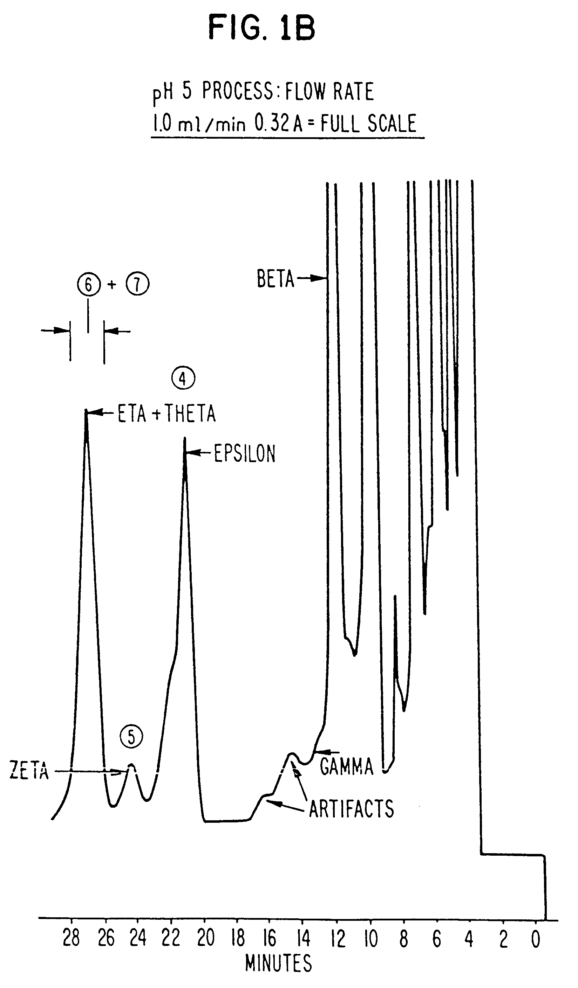

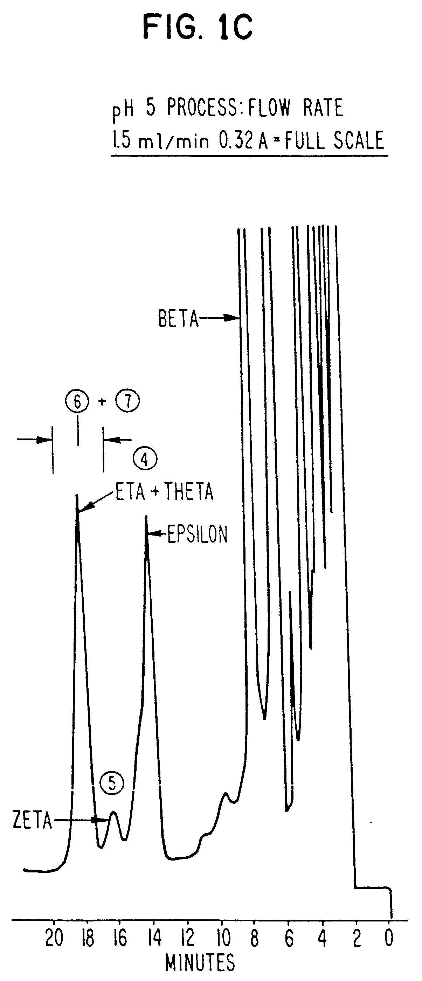

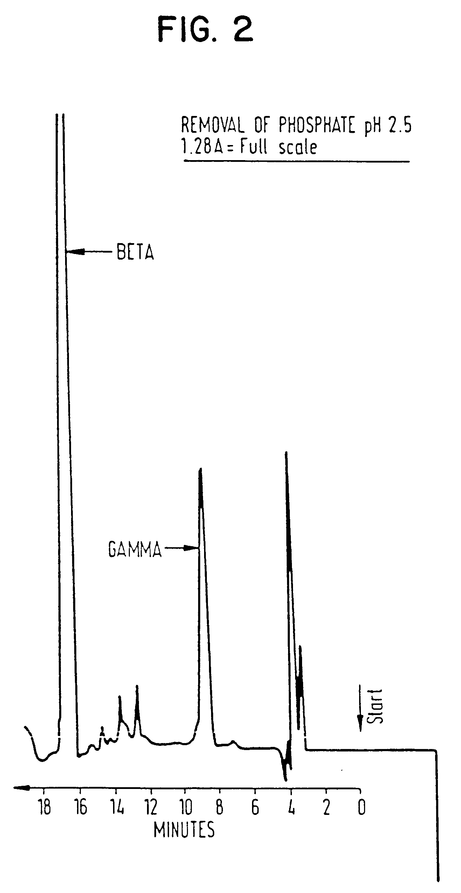

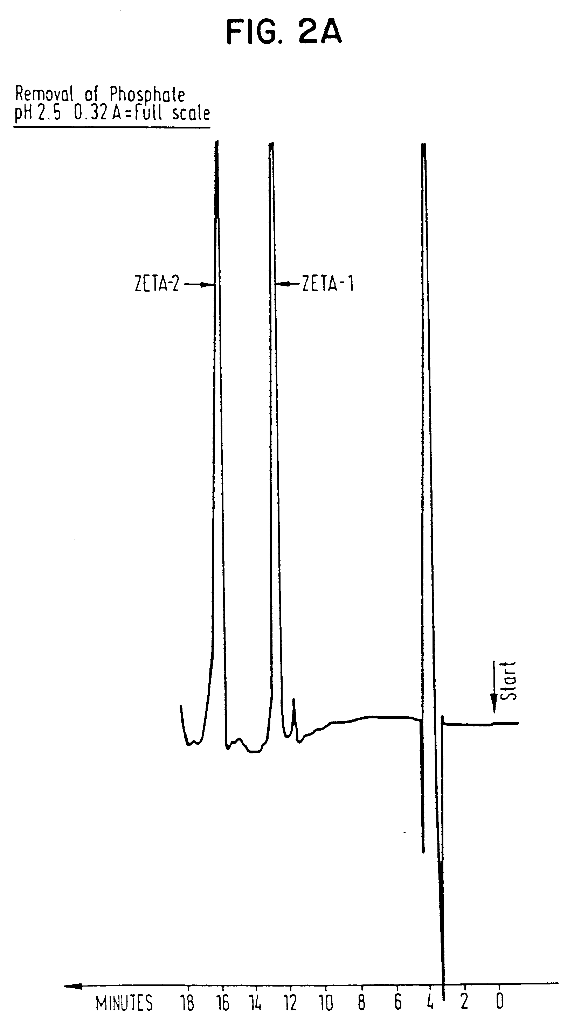

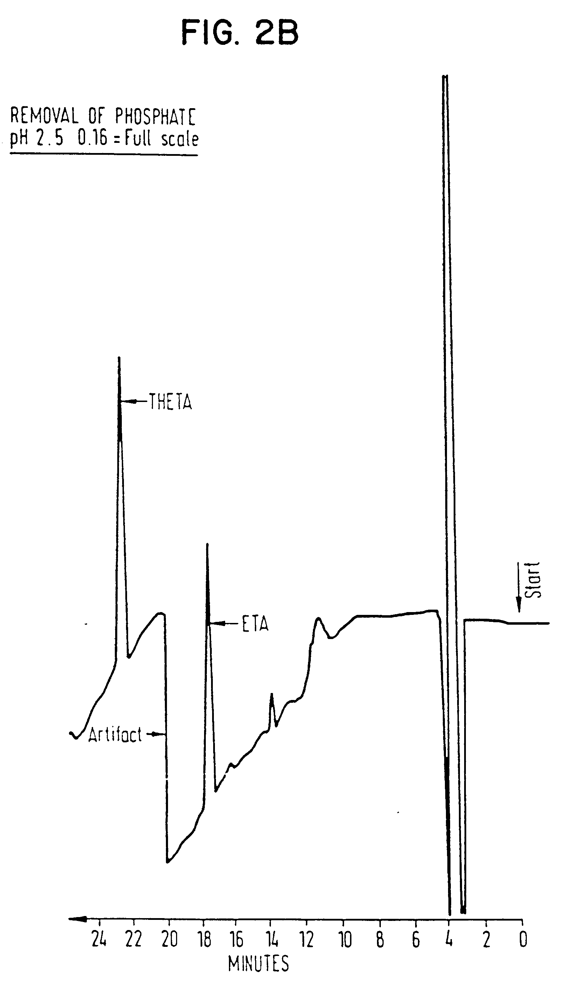

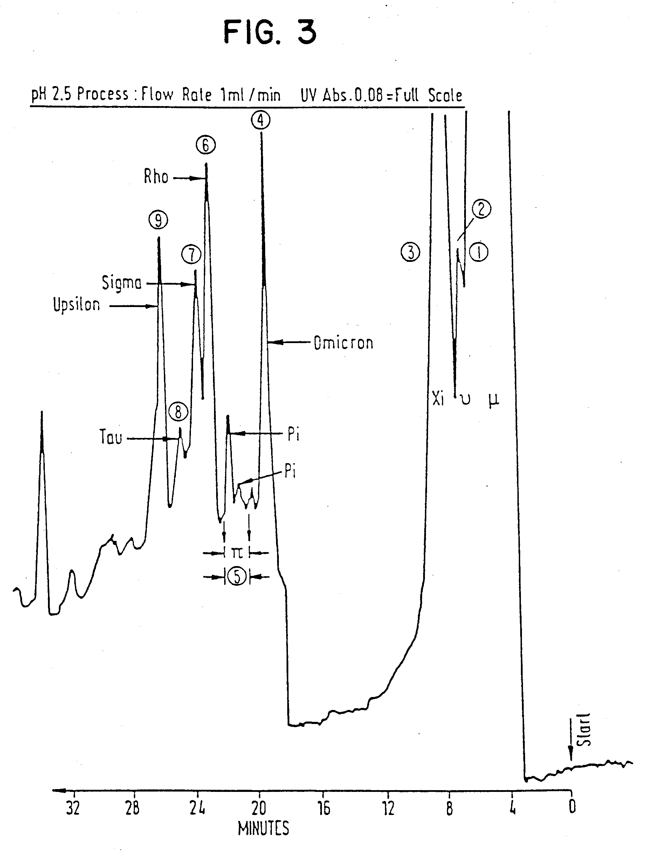

**Ultraviolet absorption measurements.** An alternative, and possibly preferable, measurement of the identity of the effluents is provided by the ultraviolet absorption profile (hereinafter "UVAP") characteristics of the range of effluents. As discussed below, I have found that the shape of the UVAP remains recognizable, despite some variations in the extraction procedure, such as a change in throughput rate. Accordingly, it is possible to identify particular materials as they come off the column by the shape of the UVAP at that point. Figure 1 shows such a UVAP for the effluents of the pH 5 process. (Figures 2 and 3 show similar data for other processes described herein.) Figures 1A through 1C show the same profile for different flow rates, showing that the profile remains recognizably the same even though the flow rate is changed. In the case of solvents whose refractive index is difficult to measure with accuracy, such as acetonitrile, the UVAP is a preferable means of identifying an effluent of interest, and when coupled with elution time it provides what I consider the best mode of for identifying an effluent of interest.

**Retention time.** In monitoring ultraviolet absorption, it is advantageous simultaneously to monitor "retention time" and also the machine's visual display of the solvent concentration at the column input. As indicated above, the display figures for concentration can by calculation be converted into estimated actual concentration data. "Retention time" is a measure of elution time. "Retention time" is used hereinafter to mean the time elapsed from the beginning of an HPLC run to the time that a particular effluent fraction passes the machine's ultraviolet detector unit on the way to the output. This occurs about 48 seconds before the effluent actually passes out of the column, here, because the volume of tubing between the detector and the output is approximately 0.8 ml and the flow rate is approximately 1 ml/min.

Retention time changes for a particular column as it "ages" through repeated use. The retention times stated here are for a new column, used at a temperature between 22°C and 24°C. As a practical matter, it is possible to calibrate an aged column for the processes used herein by running a labelled known material through it. For example, I use purified commercial Tyr-Gly-Gly for this purpose, because it reproducibly elutes approximately 0.8 to 1.2 minutes before one of the principal materials of interest discussed hereinafter (an amplifier hereinafter referred to as Beta 1.11).

### B. pH 5 process

A description of the first acetonitrile purification process (the pH 5 process) follows. It may be used with the preparation of any of Examples 1 to 3. There is a clear economic advantage in using the less labor-intensive preparation of Example 1.

### EXAMPLE 4 -- HPLC on Analytic Column, pH 5 Process

Material prepared in accordance with preceding Example 1 was further purified and separated by HPLC on the analytic column, as follows: First, an aqueous potassium phosphate solution was prepared (presumably, a mixture of K₂HPO₄ and KH₂PO₄) by adding 5 M KOH aqueous solution dropwise to 0.02 M reagent grade phosphoric acid aqueous solution until the pH of the solution was adjusted to pH 5.0. The solution was delivered to the Perkin-Elmer machine, along with HPLC grade acetonitrile. The machine was programmed to deliver the following three gradients:
(1) 10 minutes of 0 to 0.1% concentration of acetonitrile in phosphate solution, constant gradient;
(2) 45 minutes of 0.1% to 10.0%, linear gradient; and
(3) 10 minutes of 10.0% to 15.0%, linear gradient.
The flow rate was set at 1 ml/min. Then, 5-10 mg of material of preceding Example 1 was loaded into a Waters "mu Bondapak"™ O.S. column, and HPLC was commenced.

As effluent was collected, the ultraviolet absorption of the effluent was scanned with the machine's ultraviolet detector (full scale = 0.32 units). A plot of the absorption data is shown in Figure 1. Apparent solvent concentrations, retention times, and ultraviolet absorption were recorded. The results of the run are explained below and summarized in Table A thereafter.

Certain biologic materials extracted by the procedure (hereinafter identified by the names Beta, Delta, Zeta, and Eta) have been tested by the modulator assay methods described in the '379 patent. I found them to display amplifier activity.

For that reason, these effluent fractions are collected and set aside, and the others (hereinafter identified by the names Alpha, Gamma, Epsilon, and Theta) are discarded.

As indicated, during the elution process, the ultraviolet absorption of the effluent was scanned at 210 nm, with full scale absorbance set at 0.32 units. As shown in Figure 1, I observed a distinct doublet ultraviolet absorption peak at retention time 12-14 minutes (approximately 0.5 to 0.9% acetonitrile concentration as observed on the machine's display and approximately 0.1 to 0.2% estimated actual concentration). The material accompanying this peak is designated herein as Alpha. It has no known immunological activity. But its elution serves as an indication ("marker") that material designated herein as Beta, which I discovered to have significant amplifier activity, is about to elute.

Beta elutes approximately 3 minutes later, between approximately 15 and 20 minutes retention time. It is accompanied by a sharp, single ultraviolet absorption peak (hereinafter referred to as the Beta peak) reaching full scale. Displayed solvent concentration is 1.2 to 2.2%; estimated solvent concentration is 0.4 to 1.5%.

The material at the front edge of the Beta peak frequently contains a material hereinafter described as Beta 1.12, which I identified as the tripeptide Tyr-Gly-Gly or a derivative thereof. Sometimes, such material appears as a shoulder on the Beta peak. For example, in Figure 1, there is a small shoulder at about 15 minutes that corresponds to an effluent containing Tyr-Gly-Gly. For other examples, see Figure 1A (small peak in the trough between 21 and 22 min), Figure 1B (shoulder at approximately 11-12 min, preceding main Beta peak).

Immediately after Beta, at retention time between approximately 17 and 22 minutes, material designated herein as Gamma elutes. The Gamma material is characterized by either a distinct broad peak of ultraviolet absorption or as a shoulder on the absorption indication at the end of the elution of Beta. (Figure 1 shows Gamma as a distinct peak about 40% of full scale. In the runs of Figures 1A-1C, where a different Example 1 preparation was used, Gamma is only a shoulder on the descending end of Beta.) Like Alpha, Gamma is without known immunological activity.

A group of peaks are then observed in the retention time range of from 23 to 36 minutes. The first of these peaks (a rather low one, less than 30 or 40% of full scale) corresponds to (i.e., occurs with the elution of) biologic material designated herein as Delta, which has amplifier activity. Delta elutes in the retention time range of approximately 26-28 minutes. Solvent concentration indicated on the visual display of the machine is from 3.5 to 4.0%, while estimated actual solvent concentration is 2.8 to 3.3%. Delta does not maintain a well-fixed location in this process, and may either not come off the column at all or be buried in the next material (Epsilon). (In the runs of Figures 1A-1C, Delta did not elute as a recognizable peak.)

The next large absorption peak in the group is approximately 20% (or much more) of full scale, occurs about a minute later at retention time 27-30 minutes, and is a broad single peak or a doublet; the peak accompanies material designated herein as Epsilon. It is without known immunological activity.

The absorption peak immediately following, approximately 2 minutes later, at retention time about 29-32 minutes, is designated herein as Zeta. Zeta has amplifier activity. As indicated below, Zeta can by a further process be separated into two moieties, Zeta-1 and Zeta-2. Zeta-2 contains the entire amplifier activity of the Zeta material.

Eta immediately follows, about a minute later (retention time 30 to 33 minutes), accompanying the next peak. Eta contains an amplifier. Eta accompanies a very small broad absorption peak or a shoulder on the next and last absorption peak (Theta), which is much higher. (In Figure 1, Eta is shown as a shoulder on Theta.)

Finally, Theta, without known immunological activity, elutes at approximately 32 to 36 minutes. It accompanies a single large absorption peak. (In some runs, Eta does not come off the column separately from Theta, but instead comes off with it. This is illustrated in Figures 1A-1C.)

The foregoing information is summarized in Table A, below:

**Table A**

| HPLC Run, pH 5 Process | | | | |
|---|---|---|---|---|
| Range of Retention Times (mins.) | Concentration % | | U/V Data | Designation |
| | Display | Estim. | | |
| 12-14 | 0.5-0.9 | 0.1-0.2 | peak | Doublet Alpha |
| 15-20 | 1.2-2.2 | 0.4-1.5 | Single peak | Beta |
| 17-22 | 1.5-2.6 | 0.8-1.9 | Broad moderate peak or shoulder on end of preceding peak | Gamma |
| 26-28 | 3.5-4.0 | 2.8-3.3 | First peak of 23-36 group of peaks, low to moderate peak following no-absorption trough. May fail to appear. | Delta |
| 27-30 | 3.7-4.4 | 3.0-3.7 | Next large peak of 20% or more of full scale | Epsilon |
| 29-32 | 4.2-4.8 | 3.5-4.1 | Broad single peak or overlapping doublet | Zeta |
| 30-33 | 4.4-5.1 | 3.7-4.4 | Small broad peak or part of or shoulder on beginning of next peak (Theta) | Eta |
| 32-36 | 4.8-5.7 | 4.1-5.0 | Single large peak. May include Eta. | Theta |

### C. Acetonitrile cleanup process

The materials of Example 4 are contaminated with phosphate ions and are imperfectly purified from extraneous material (material having no known useful immunological activity). I discovered a further HPLC procedure with a different solvent system, which can remove phosphate and extraneous material. The resulting material appears to be considerably more free of extraneous material.

### EXAMPLE 5 -- HPLC Cleanup and Phosphate Removal

First, a 0.1% (v/v) aqueous trifluoroacetic acid (Mallinckrodt, Inc., Paris, Ky.) solution was prepared, and the pH of the solution was adjusted to pH 2.5 by the dropwise addition of sufficient 5 M KOH aqueous solution. The solution was delivered to the Perkin Elmer machine, along with HPLC grade acetonitrile.

The machine was programmed for a 45 minute linear gradient of 0.1% to 45% concentration of acetonitrile in the trifluoracetic acid solution. (A 25-minute run to 25% is acceptable, but 45 minutes to 45% is more conservative.) The flow rate was set at 1 ml/min. The machine was then used for any one of the following three separate processes.

### a. Beta Run

Beta fractions from approximately 4 procedures of Example 4 are pooled and loaded into a "mu Bondapak"™ O.S. column, as above; and HPLC is commenced. The effluents are scanned with the ultraviolet absorption detector, as in the preceding example. Full scale is set at 1.28 absorption units.

Contaminating Gamma material elutes as shown in Table B, below, and is discarded. Material hereinafter designated as Amplifier Beta-1.0 elutes as shown in Table B. Tyr-Gly-Gly, which as discussed below is a material having immunological activity, is frequently, but not always, observed as an effluent associated with a small peak or shoulder on the front edge of the Beta peak in the pH5 acetonitrile process; sometimes, it is found pre-peak. (See discussion following Example 4.)

### b. Zeta Run

The same procedure is repeated with the Zeta material of Example 4. Zeta fractionates into two moieties -- hereinafter designated Zeta-1 and Zeta-2. The first is without known immunological activity. The second has amplifier activity. Zeta-1 and Zeta-2 elute as shown in Table B.

### c. Eta Run

The same procedure is repeated with the Eta material of Example 4. Material hereinafter designated as Amplifier Eta-1 elutes as shown in Table B. Contaminating Theta material, previously mixed with the Eta material, elutes later and is discarded.
Unfortunately, Delta has not been recovered by use of this procedure; it may be destroyed by the trifluoroacetic acid. The foregoing results are summarized in Table B, below. Figures 2, 2A, and 2B show the UVAPs associated with these three procedures.

**Table B**

| HPLC Cleanup | | | | |
|---|---|---|---|---|
| | Range of Retention Times (mins.) | Concentration % | | Designation |
| | | Display | Estim. | |
| 1. | 8-11 | 8-11 | 4.8-7.8 | Gamma |
| | 15-18 | 15-18 | 11.8-14.8 | Beta-1.0 |
| 2. | 11-14 | 11-14 | 7.8-10.8 | Zeta-1 |
| | 15-19 | 15-19 | 11.8-15.8 | Zeta-2 |
| 3. | 18-20 | 18-20 | 14.8-16.8 | Eta-1 |
| | 23-26 | 23-26 | 19.8-22.8 | Theta |
| Note: These are three different procedures, using three different starting materials (Beta, Zeta, and Eta, respectively.) | | | | |

### D. pH 2.5 process

A description of the second acetonitrile purification process (the pH 2.5 process) follows.

### EXAMPLE 6 -- HPLC on Analytic Column, pH 2.5 Process

Material prepared in accordance with preceding Example 3 was further purified and separated by HPCL on the analytic column as follow: First, an aqueous potassium phosphate solution was prepared as in preceding Example 4, except that the pH was adjusted only to 2.5. The solution was delivered to the Perkin-Elmer machine, along with HPLC grade acetonitrile.

The machine was programmed to deliver the following three gradients: (1) 10 minutes of 0.1% to 5% concentration of acetonitrile in phosphate solution, gradient curve 4 (Perkin-Elmer LC75 program); (2) 30 minutes of 5% to 20%, gradient curve 0.2 (Perkin-Elmer LC75 program); (3) 10 minutes of 20%, constant gradient. The flow rate was set at 1 ml/min.

Then, 5-10 mg of fluram-reactive "S" fractions associated with the major fluram-reactive peak of Example 3 was loaded into a "mu Bondapak" O.S. column and HPLC was commenced.

Monitoring of ultraviolet absorption detector (full scale = 0.08 units) shows nine or more peaks. The results of the run are summarized in Table C. Ultraviolet absorption detection at 210 nm is plotted in Figure 3. Materials extracted by the procedure (hereinafter designated by the names Pi, Sigma, and Upsilon) have been tested by the methods of the '379 patent and have been found to display amplifier activity. These effluent fractions are collected and set aside. The other fractions (hereinafter designated Mu, Nu, Xi, Omicron, Rho, and Tau) are discarded.

The data relating to the elution of these amplifier materials is summarized below in Table C.

**Table C**

| HPLC Run, pH 2.5 Process | | | | |
|---|---|---|---|---|
| Retention Times (Mins.) | Concentration % | | Ultraviolet Data | Designation |
| | Display | Estim. | | |
| 0-18 | 0.1-12.6 | 0.1-11.3 | 4 peaks | Mu, Nu, Xi Omicron |
| 19.6-22.4 | 13.5-14.5 | 12.0-13.5 | 2 small peaks | Pi |
| 22.6-23.4 | 14.8-15.0 | 13.8-13.9 | First large peak of a doublet with Sigma | Rho |
| 23.6-25.0 | 15.3-15.8 | 14.0-14.5 | Large second peak of Rho doublet | Sigma |
| 25-28 | 15.8-16.7 | 14.5-15.6 | Indistinct trailing peak on end of Sigma | Tau |
| 28.2-29.2 | 16.8-17.1 | 15.8-16.0 | Next large peak after Tau | Upsilon |

### E. Further Purification Processes

I have developed further refinements have been that further purify the products of these processes, and remove additional extraneous material. The Beta material described above has been further fractionated and refined by these techniques, and the resulting products have become sufficiently purified to permit amino acid analysis thereof. A description of the processes follows.

### EXAMPLE 6A -- Ethanol Purification of Beta

The material developed from the pH 5 phosphate gradient of Example 4, and designated as Beta (see Table A), was dried in a vacuum evaporator without heat. The material was reconstituted in 0.1% trifluoroacetic acid to approximately 100 microliter per 200 mg of fluram-reactive starting material. A Perkin-Elmer preparative column was used for HPLC; a 3.5 cm x 28 cm column was packed with octadecylsilane, and the reconstituted material was introduced. A flow rate of 6.0 ml/min was set.

The machine was programmed to deliver the following gradients:
(1) 30 minutes of ethanol in water, from 0% ethanol to a 50.0% concentration, linear gradient;
(2) 5 minutes, linear gradient, from 50.0% to 0% ethanol; and
(3) 10 minutes constant gradient of 100% water.

Absorbance was monitored at 254 mm, 0.1 Absorbance Units = Full Scale. Three major peaks were observed, which respectively eluted at 10.4-10.6 min, 14.0-16.0 min, and 18.0-19.0 min. The biological activity was assayed and found to reside almost entirely in the material associated with the middle peak, which is designated herein as Beta-1.1. The last peak was found to be associated with material that was mainly phenylalanine and contained approximately 90% of the starting material used. The first peak was lower than the second and third, in terms of absorbance units. The second and third peaks varied in relative height from preparation to preparation.

Based on refractive index measurements, the ethanol concentration of the effluent of the second peak was found to be from approximately 0.1% to approximately 0.4%.

An amino acid assay of the material associated with the Beta-1.1 peak was made, with the following normalized results:

| | |
|---|---|
| Asx | 2 |
| Thr | 1 |
| Ser | 1 |
| Glx | 1 |
| Pro | 2 |
| Gly | 4 |
| Ala | 2 |
| Val | 1 |
| Ile | 1 |
| Tyr | 1 |
| Lys | 3 |
| His | 1 |
| Arg | 1 |
| | 2̅3̅ |

I decided that still further purification of the materials of the foregoing process would be desirable, to ascertain whether one or more immunologically active materials were present in which there were substantially fewer peptide groups. I devised an acetonitrile HPLC process that, to date, resulted in the extraction of two molecules having amplifier activity: a material containing the dipeptide Tyr-Gly and a material containing the tripeptide Tyr-Gly-Gly. The process is in some ways similar to the "cleanup" process of Example 5; but a different column is used, the trifluoroacetic acid solution is twice as dilute, and the gradient is different. The Zorbax™, Dupont column used here comes packed with a Dupont material containing octadecylsilane groups chemically bonded to silica particles.

### EXAMPLE 6B - Dilute Acetonitrile Purification of Beta 1.1

Beta-1.1 material of Example 6A was injected into an ODS HPLC column (Zorbax™, DuPont), 1 ml/min flow rate, 25°C. The solvent system was (1) 100% acetonitrile (CH₃CN), HPLC Grade, and (2) 0.05% trifluoroacetic acid aqueous solution, HPLC Grade, pH 2.5. The following linear gradients were used:

| Time segment | Cumulative time | % CH₃CN |
|---|---|---|
| 0 | 0 | 0 |
| 15 | 15 | 6 |
| 30 | 45 | 40 |
| 5 | 50 | 0 |
| 1 | 51 | 0 |

A fraction of interest, hereinafter designated as Beta-1.11, eluted at approximately 23.8 to 24.8 min, for a new column. It was associated with a sharp, narrow peak of UV absorption at 210 nm; this peak is approximately the fourth absorption peak observed (some variability existing). The material was lyophilized and reconstituted in normal saline, and set aside for further use. As shown below, Beta-1.11 contains Tyr-Gly, a dipeptide having amplifier activity.

Another fraction of interest, eluted from this material on this column, but somewhat irregularly. When it eluted, the retention time was approximately 22.8-23.2 min. It was associated with a distinct peak of ultraviolet absorption at 210 nm, just before the peak associated with Beta-1.11. The material was lyophilized and reconstituted in normal saline, and set aside for further use. As shown below, this material, hereinafter referred to as Beta-1.12, contains Tyr-Gly-Gly, a tripeptide having amplifier activity.

Other moieties found present in effluents of this process were Gly-Gly, Ser-Phe, and Ala, along with possibly other amino acid residues as well. None of these other moieties appeared to possess immunological activity, as assayed by DH test.

To avoid confusion between the two moieties of interest, particularly in the case of aged columns, I have found it advantageous to run purified commercial Tyr-Gly-Gly through the column as a marker. As indicated above, such Tyr-Gly-Gly elutes reproducibly 0.8 to 1.2 min before Beta-1.11 (approximately the same zone as Beta-1.12).

A Tyr-Gly-Gly purification procedure is now described which I devised to eliminate pyrogens, endotoxins, and other contaminants present in the commercial product. I did so to provide a synthetically-derived Tyr-Gly-Gly product for use as a marker in the foregoing process and also for use as an amplifier for human subjects. The resulting product, which I believe has not been hitherto described, appears to be free of all toxic or harmful contaminants usually found in the commercial product.

### EXAMPLE 6C - Purification of Tyr-Gly-Gly

1 microgram (ug) of synthetic, crystalline Tyrosylglycylglycine (Sigma Chem. Co., St. Louis, Mo.) is dissolved in 1 microliter (ul) of normal saline. The solution is mixed with 99 ul of 0.05% trifluoroacetic acid (TFA) in water. 50 ul of this mixture is injected into a fresh reverse-phase HPLC analytic column (Zorbax™, Dupont, 4.6 mm x 25 cm) and eluted with the following combination of linear gradients:
- Solvent A: = 100% acetonitrile
- Solvent B: = 0.05% trifluoroacetic acid in water

| Time | % A to % B | |
|---|---|---|
| Init. | 0 | 100 |
| 15 min. | 6 | 94 |
| 45 min. | 40 | 60 |
| 50 min. (end) | 0 | 100 |

The elution curve absorbance at 210 A (0.05 O.D. units full scale) shows a sharp and narrow peak, much higher than any other peaks, at approximately 21.5 to 22.7 minutes. This elutant is TGG, substantially free of contaminants. (Note: the 22.7 min figure is for a fresh column.)

The material associated with the peak is recovered, lyophilized, and made up in sterile normal saline.

I then subjected the Beta-1.11 material to amino acid sequencing procedures. Using standard techniques, I determined that Amplifier Beta-1.11 contains Tyr and Gly, which come off in that order. Therefore, I identified the material as containing the dipeptide Tyr-Gly, which I found was immunologically active, as discussed below. (A protein/peptide sequencer automatically removes one amino acid at a time from the N-terminal end of a protein or peptide for determination of amino acid sequence.) I also detected Tyr-Gly-Gly in this material, which I found was immunologically active, as is discussed below. Small amounts of Ile and Lys were also detected. Those may be artifacts of the procedure, or may indicate small amounts of Ile/Lys components in a molecule or molecules present in relatively low concentrations in the product (such as, hypothetically, Tyr-Ile-Lys, Tyr-Gly-Lys, Tyr-Gly-Gly-Ile). I presently believe that the Ile/ Lys material is simply an artifact or a nonsignificant component.

I also subjected the Beta-1.12 material to amino acid sequencing procedures and found that it contained Tyr-Gly-Gly. As discussed below, I found this tripeptide to be immunologically active.

I have not ascertained whether isomeric forms are present (such as D-aminoacid forms), whether the Tyr-Gly and Tyr-Gly-Gly materials of Beta-1.11 and 1.12 have other groups bound to them (e.g., methyl, acetyl, amide), or whether complexes with metal ions (such as, possibly, Zn++, Ca++, Fe++, Mn++, or Mg++) are present, and if so whether the different forms, if any, possess different immunological activity. As indicated below, certain immunological assay data suggests that Beta-1.11 and Beta-1.12 are in some way different from chemically manufactured Tyr-Gly and Tyr-Gly-Gly, respectively, because the products extracted from natural (i.e., human) material appear to have greater immunological activity than the purified peptide chemicals. (The purified chemicals do not have methyl, acetyl, etc. substitutions and they are not complexed with Zn++ or other ions. They may contain D-aminoacid residue isomers.)

There is reason to infer that the naturally occurring Beta-1.11 and 1.12 amplifier products are present in the human or animal body in the form of a complex of (X)M++(Y), where (X) and (Y) are each selected from the group consisting of Tyr-Gly, Tyr-Gly-Gly, or a derivative thereof, and where M++ is Zn++ or another divalent metallic ion such as Ca++, Fe++, Mn++, or Mg++. There is a negative site on the Tyr group that may bind to the M++ ion; there is also a C-terminal negative site on the last Gly that may so bind. There may well also be a mixture of such complexes.

I have done some analysis of the ratio of Tyr-Gly and Tyr-Gly-Gly components in human materials, by radioiodinating the Tyr groups and then separating the Tyr-Gly and Tyr-Gly-Gly moieties by gel electrophoresis. If there were only a (Tyr-Gly)M++(Tyr-Gly-Gly) complex, one could anticipate a constant 1:1 ratio of the two moieties. But that does not occur; instead, the proportions observed varied from sample to sample. This suggests the presence of several different materials or complexes -- such as (Tyr-Gly)M++(Tyr-Gly), (Tyr-Gly)M++(Tyr-Gly-Gly), and (Tyr-Gly-Gly)M++(Tyr-Gly-Gly) -- in varying proportions, depending on as yet unidentified physiological parameters.

From the foregoing amino acid sequencing data, in conjunction with biological assay data described below, I conclude as follows: The biologically most significant component or components of the Beta material described above may be further characterized as containing the amino acids Tyr and Gly, in a 1:1 or 1:2 ratio, and as not containing substantial amounts of any other amino acid. The Tyr-Gly and Tyr-Gly-Gly present in the Beta materials may exist as a mixture of fixed or variable proportions; either or both molecules may be complexed with metal ions (such as Zn++, Ca++, Fe++, Mn++, or Mg++); both may be complexed together, with the possible addition of metal ions. The materials eliminated from the final materials, such as Phe and Gly-Gly, do not possess intrinsic immunological activity, but they may co-act with the Tyr-Gly and Tyr-Gly-Gly materials to enhance their stability or immunological activity.

### F. HPLC Yield Data

The foregoing series of processes begin with a leukocyte dialysate containing an extract from approximately 10¹⁰ leukocytes. This results in approximately 300 mg of fluram-reactive material of Example 3. This in turn yields approximately 500 ng of Beta-1.0. This in turn yields approximately 3 or 4 ng of Tyr-Gly and Tyr-Gly-Gly each. A quantity of 0.1 pg of Tyr-Gly, which is approximately one clinical dosage unit, as discussed below, is derived from approximately 4 x 10⁵ leukocytes. (However, the same starting material also yields approximately the same amount of the tripeptide Tyr-Gly-Gly of interest.)

### III. Biological Assay Data

Additional biological assay methods have been developed, which were not described in the '379 patent. The '379 patent describes assaying amplifier material by the enhanced DH response in normal subjects when they are re-exposed to an antigen. In addition, I have now found the following assay methods advantageous:
(1) antigen-induced enhanced "leukocyte inhibitory factor" ("LIF");
(2) augmented production of interleukin-2 ("IL-2"), stimulated by mitogen, antigen, or alloantigen;
(3) enhanced generation of cytotoxic cells to Raji cells;
(4) augmented production of gamma-interferon, stimulated by mitogen or antigen; and
(5) enhanced expression of high-density receptors for IL-2.

I believe that criterion (4) is secondary to the augmented production of IL-2 of criterion (2). Beta and Zeta-2 display at least three out of the five new assay criteria as well as the enhanced DH response of the '379 patent. Beta displays all five; Zeta-2 has not yet been tested with the LIF assay. Delta enhances mitogen-induced IL-2 production. Eta enhances generation of cytotoxic cells to Raji cells. Delta, Eta, Pi, Sigma, and Upsilon display the enhanced DH response of the '379 patent, but have not been otherwise tested as yet with the five newer assay procedures.

The protocol for the enhanced DH assay is given in the '379 patent. The LIF assay is described in Gottlieb et al., Modulation of Human T Cell Production, J. Immunology 132:256-260 (Jan. 1984), at p. 257. Protocols for the other assays are believed to be known to those skilled in this art.

As stated above, it has been shown that the materials of this invention have amplifier activity. This was done by means of the DH assay procedure of the '379 patent, as indicated in the following examples.

### EXAMPLE 7 -- DH Assay of Beta-1.0

Serial dilutions of Beta-1.0 of preceding Example 5 were made from a solution containing the amplifier material derived from 4 x 10⁸ buffy coat leukocytes in 1 ml of aqueous saline solution. 0.05 ml of tetanus toxoid was diluted from 1/10 to 1/40 so as to elicit a small (preferably slightly less than 5 x 5 mm) skin reaction from the patient, and 0.1 ml of the diluted Beta-1.0 preparation was added. The patient was subcutaneously injected with several different dilutions of Beta-1.0, and also with an equal quantity of tetanus toxoid (TT) without any Beta-1.0 added thereto. Two approximately perpendicular diameters of each responding skin site on the man's arm were measured at the times indicated below. ("TT + _" refers to TT and a dilution of Beta to the concentration indicated; "TT" alone is TT without Beta-1.0.)

At 5 hours, the respective responses to TT + 10⁻⁸, TT + 10⁻⁹, and TT were 14 x 14 mm, 19 x 14, and 3 x 3. At 24 hours: 20 x 24 mm, 19 x 23, 14 x 12.

### EXAMPLE 7A - DH Assay of Amplifier Beta-1.11

Example 7 was repeated with serial dilutions of Amplifier Beta-1.11, beginning with 1 microgram per microliter of the product of Example 6B. It is estimated that 100 picograms of the product of Example 6B is the amount of Amplifier Beta-1.11 that can be derived from approximately 3 x 10⁸ buffy coat leukocytes. It is also estimated that the M.W. of Amplifier Beta-1.1 is approximately 239. Hence, 1 microgram/microliter is approximately a 4 mM solution. Dilutions to 4 femtomolar (fM) and 0.4 fM were prepared.

After following the procedure described in Example 7, it was observed that at 5 hours, the respective responses to TT + 0.1 ml 4fM, TT + 0.1 ml 0.4 fM, and TT were 7 x 8 mm, 9 x 9, and 3 x 5. At 24 hours: 18 x 21 mm, 19 x 19, 14 x 15.

### EXAMPLE 7B - DH Assay of Amplifier Beta-1.12

Example 7 was repeated with serial dilutions of Amplifier Beta-1.12, beginning with 1 microgram per microliter of the product of Example 6C. It is estimated that 100 picograms of the product of Example 6C is the amount of Amplifier Beta-1.11 that can be derived from approximately 3 x 10⁸ buffy coat leukocytes. It is also estimated that the M.W. of Amplifier Beta-1.1 is approximately 295. Hence, 1 microgram/microliter is approximately a 3 mM solution. Dilutions to 3 fM and 0.3 fM were prepared.

After following the procedure described in Example 7, it was observed that at 5 hours, the respective responses to TT + 0.1 ml 3fM, TT + 0.1 ml 0.3 fM, and TT were 7 x 8 mm, 9 x 9, and 3 x 5. At 24 hours: 18 x 21 mm, 19 x 19, 14 x 15.

In the case of Beta-1.12, it is not certain whether the DH response is to the Beta-1.12 per se (i.e., to material essentially consisting of Tyr-Gly-Gly per se) or to a Beta-1.11 (i.e., material consisting essentially of Tyr-Gly) metabolite of Beta-1.12. It is known that pentapeptides (enkephalins) containing a Tyr-Gly-Gly amino acid residue sequence are degraded in vivo by the action of dipeptidylaminopeptidase, which hydrolyzes the enkephalin (i.e., H₂N-Tyr-Gly-Gly-Phe-Met-OH) by cleaving its Gly-Gly amide bond. (See, e.g., Schwartz, Malfroy, and Baume, Biological inactivation of enkephalins and the role of enkephalin-dipeptidyl-carboxypeptidase ("enkephalinase") as neuropeptidase, 29 Enkephalin Metabolism 1715, 1716 (1981), which cites and summarizes a number of studies in this field.) Hence the apparent immunoamplificatory effect of the Tyr-Gly-Gly might be attributable to Tyr-Gly as a metabolite of Tyr-Gly-Gly rather than to the Tyr-Gly-Gly itself. (For example, it is known that hetacillin converts to ampicillin once in the human body, and therefore produces a similar effect. That is, the metabolite of hetacillin acts as an antibiotic.)

On the other hand, both molecules may have intrinsic immunoamplificatory effect. For example, the receptor site for this lymphokine could be associated with the Tyr-Gly portion of the molecule, and the additional Gly amino acid residue may have a neutral effect on reception and biological activity. It is also possible that Tyr-Gly-Gly interacts with a different receptor site to have the same biological effect as Tyr-Gly. Alternatively, a complex of both peptides, perhaps through a trace metal (such as Zn++, Ca++, Fe++, Mn++, or Mg++) may be required. This is a question calling for further work, such as in vitro tests of Tyr-Gly-Gly in the presence of a suitable aminopeptidase inhibitor, to prevent cleavage of the Tyr-Gly amide bond in Tyr-Gly-Gly to produce Tyr-Gly. In any event, whether Tyr-Gly-Gly operates per se or as a metabolite of Tyr-Gly, Tyr-Gly-Gly clearly can be used to enhance amplifier activity (as hetacillin may be used to produce an ampicillin-like effect) and is thus considered within the instant invention.

This question is complicated further by an additional factor. I made assays with Tyr-Gly and Tyr-Gly-Gly preparations that I prepared by purifying commercial, synthetic peptide products. I purified the Tyr-Gly and Tyr-Gly-Gly preparations by removing pharmaceutically unacceptable (potentially toxic) contaminants from them. The purified commercial peptides show lower immunological activity (for the same weight of active chemical) than do the corresponding preparations made from human leukocyte dialysates. If the human-derived products were identical to the purified commercial peptide chemicals, this difference in activity should not occur.

Therefore, I conclude that they are not identical. The commercial products may in whole or part be different isomeric forms from the biological products. The biological materials may be complexed, as discussed above, for example, with trace metal ions, such as Zn++. The biological materials may be more stable than the synthetic chemical materials, in vivo, because of chemical differences. While the purified synthetic products are simply the dipeptide or tripeptide, per se, rather than derivatives thereof, the biological products may contain substitutions such as a methyl, acetyl, or amide for an H; or other molecular variations may occur, which are not detected by the analytic procedures utilized here.

However, while the products derived from commercially available chemicals appear to be less active biologically, they are considerably cheaper to prepare and may possibly produce more repeatable results. Consequently, there may be a tradeoff of factors leading to a conclusion that the chemically-derived products are biologically adequate, even though perhaps suboptimal, and are therefore the basis of a preferred commercial embodiment.

I return now to the DH assay data for the other amplifiers of this invention.

### EXAMPLE 8 -- DH Assay of Delta

The procedure of Example 7 was repeated with Delta of Example 4. At 6 hours, the respective responses to TT + 10⁻⁷ and TT were 19 x 18 mm, 13 x 11. At 23 hours: 28 x 29 mm, 7 x 6.

### EXAMPLE 9 -- DH Assay of Zeta-2

The procedure of Example 7 was repeated with Zeta-2 of Example 5. However, purified protein derivative of tuberculin ("PPD") was substituted for TT. At 12 hours, the respective responses to PPD + 10⁻⁶, PPD + 10⁻⁷, PPD + 10⁻⁸, and PPD were 1 x 1 mm, 15 x 22, 14 x 11, and 1 x 1. At 27 hours: 2 x 2 mm, 22 x 24, 15 x 15, and 3 x 3.

### EXAMPLE 10 - DH Assay of Eta

The procedure of Example 7 was repeated with Eta of Example 5. At 4 hours, the respective responses to TT + 10⁻³, TT + 10⁻⁴, TT + 10⁻⁵, TT + 10⁻⁶, and TT were 2 x 3 mm, 7 x 7, 2 x 3, 3 x 3, and 2 x 2. At 13 hours: 8 x 11 mm, 16 x 20, 16 x 15, 4 x 5, and 6 x 4.

### EXAMPLE 11 -- DH Assay of Pi

The procedure of Example 7 was repeated with Material Pi of Example 6. At 6 hours, the respective responses to TT + 10⁻⁷, TT + 10⁻⁸, TT + 10⁻⁹, and TT were 8 x 10 mm, 12 x 11, 7 x 8, and 2 x 3. At 21 hours: 12 x 12 mm, 21 x 15, 15 x 16, and 14 x 13.

### EXAMPLE 12 -- DH Assay of Sigma

The procedure of Example 7 was repeated with Material Sigma of Example 6. At 4 hours, the respective responses to TT + 10⁻⁷, TT + 10⁻⁸, TT + 10⁻⁹, and TT were 12 x 11 mm, 13 x 12, 4 x 2, and 4 x 2. At 8.5 hours: 17 x 15 mm, 19 x 20, 5 x 4, and 5 x 4.

### EXAMPLE 13 -- DH Assay of Upsilon

The procedure of Example 7 was repeated with Material Upsilon of Example 6. At 5.5 hours, the respective responses to TT + 10⁻¹ and TT were 16 x 13 mm, 10 x 8. At 9 hours: 15 x 25, 13 x 10. At 11 hours: 25 x 23, 12 x 15.

### IV. Human Tests

The effectiveness of the amplifier material of this invention in amplifying human immune system response has been tested in a number of men suffering from AIDS or ARC. All amplifier materials used in this work were free of endotoxin as detected by the Limulus assay (M.A. Bioproducts, Rockville, Md.). The following examples illustrate these tests. (As used hereinafter, the term "T-helper cell" includes cells designated as T4⁺, CD4⁺, Leu3⁺, and T4.)

The inventor has ascertained by empirical means that an effective dosage amount of these products, in the procedures described hereinafter, is that derived from 400,000 leukocytes, purified by the method of Example 5 and dispensed in 0.1 ml of normal sterile saline solution. (This dosage amount, that derived from 4 x 10⁵ leukocytes, is frequently referred to hereinafter as "**one standard dose of Beta.**")

### EXAMPLE 14 -- Multiple Doses of Beta-1.0 With Transfusion

An AIDS patient, DT, with Kaposi's sarcoma was given doses of Beta-1.0 together with transfusions of isologous leukocytes available from DT's identical twin brother (a normal, disease-free person). DT also received such transfusions without Beta-1.0.

An initial transfusion of approximately 1.0 x 10¹⁰ isologous leukocytes (without Beta-1.0) produced a moderate restoration of DT's proliferative response to phytohemagglutin (PHA). Within 13 days the response declined to baseline levels; there was no alteration in the ratio of circulating helper lymphocytes to suppressor lymphocytes (T4/T8 ratio).

Ten days after the initial transfusion, DT was given a single standard dose of Beta-1.0 (as stated above, the dose derived from 400,000 leukocytes). No effect was observed on DT's PHA response.

A cycle of treatment comprised of a second isologous leukocyte transfusion (again, the same number of leukocytes) followed at 24, 48, and 71 hours by subcutaneous Amplifier Beta doses derived from 400,000, 4,000,000, and 400,000 leukocytes, respectively (i.e., 1, 10, and 1 standard doses). A significant increase in DT's PHA response followed. There was also an increase in the T4/T8 ratio, resulting from an absolute increase of T4+ cells and a decrease in T8+ cell numbers. After approximately one month, these parameters of immune system response declined to approximately their former level.

A third transfusion similar to the first (no Beta) was given. No effect on PHA response or T4/T8 ratio was observed.

While these studies were made, parallel studies of IL-2 production were made. Initially, no IL-2 production was observed in response to PHA. This correlated with the patient's low proliferative response to mitogen. The initial leukocyte transfusion did not affect this parameter. After the second transfusion (of both leukocytes and Beta-1.0), PHA induced significant levels of IL-2.

### EXAMPLE 15 -- Multiple Doses of Beta-1.0: Group 1

The members of a group of 15 patients with AIDS received one standard dose of Beta-1.0 once every month until three doses were given (three months). Of these 15 patients, six had candida infections (oral candidiasis), and 12 had Kaposi's Sarcoma.

Clinical symptoms were monitored. No decrease in weight was observed. No toxicity to Beta was observed.

A significant decrease in candida infection was observed as a result of treatment, in three-quarters of the patients completing the protocol.

Skin test sensitivity (DH test) to tetanus toxoid was noticeably enhanced, and returned to an approximately normal level in 47% of the subjects. Since, according to the Walter Reed Classification of Severity of AIDS/ ARC (see 314 New Eng. J. Med. 131 (1986)), candida infection and loss of skin test sensitivity are signs of far advanced immunodeficiency, the effectiveness of Beta-1.0 in reversing these symptoms is medically significant.

Mitogen-stimulated lymphocyte proliferation increased with each successive dose. Mitogen-stimulated IL-2 production increased in at least 60% of patients. Response to pokeweed mitogen (PWM) increased for those patients having more than 50-100 T4 cells per mm³ remaining.

### EXAMPLE 15A -- Multiple Doses of Beta-1.0: Group 2

The members of a group of 14 patients with AIDS received one standard dose of Beta-1.0 every two weeks for six doses (approximately three months). Of these subjects, six had candida infections. Of the 14 patients, 11 had Kaposi's Sarcoma.

Clinical symptoms were monitored. Eleven of the 14 patients gained weight. An average weight gain of 4.4 lb occurred in these 11. No toxicity to Beta-1.0 was observed. Serum uric acid levels fell. Creatine phosphokinase levels fell. Since high levels of uric acid and creatine phosphokinase reflect tissue breakdown characteristic of AIDS, lowering of the levels of these substances and reversal of weight loss suggests significant clinical improvement.

Skin test sensitivity to tetanus toxoid returned in 57% of subjects. Candida infection was totally cleared in three subjects and decreased in another.

Mitogen-stimulated lymphocyte proliferation increased. Mitogen-stimulated IL-2 production increased in 60% of patients after two doses of Beta-1.0; and in all those patients having more than 50-100 T-helper cells/mm³ remaining, after two doses of Beta-1.0.

Response to pokeweed mitogen (PWM) increased for those patients having more than 50-100 T-helper cells/mm³ remaining. A small increase to PWM appeared after the second dose with those patients having fewer than 50-100 T-helper cells/mm³ remaining, and slowly increased following the next two doses.

There was also a slowing of the rate of destruction of T-helper cells during the treatment with Beta-1.0. For example, untreated patients with ARC typically lose T-helper cells at the rate of approximately 13.4 cells/month. For those of the above ARC patients who received Beta-1.0 on a monthly basis (Example 15), the rate of T-helper cell loss was 7.2 cells/month, while for those who received it every two weeks (Example 15A) the rate of T-helper cell loss was 4.2 cells/month. This data indicates that Beta-1.0 slows the rate of T-helper cell destruction typical of ARC. The retarding of destruction is proportional here to the dosage.

### EXAMPLE 15B -- Multiple Doses of Beta-1.0: Group 3

Five patients, three with AIDS (RB, JB, and RG) and two with ARC (WW and CM) were treated with Beta-1.0 over a period of approximately a year or more. (One standard dose intradermally every two weeks.)

Skin test sensitivity returned completely in three subjects and partially in one (RB, JB, WW, and CM). Candida infection improved in the two patients (RB and CM) initially having it and it did not appear in the others. The percentage of T-helper cells increased transiently in four patients (RB, RG, WW, and CM).

Three patients gained substantial weight (RB, WW, and CM). PHA-stimulated lymphocyte proliferation increased in all five, PWM response in four (RB, RG, WW and CM), IL-2 production in three (RB, WW, and CM).

These tests indicate that Beta-1.0 has a ppositive effect on the T-helper cell population of the human body and is useful in improving human immune response characterized by a T-helper cell defect. Doses of Beta appear to partially restore the functioning of a defective subset of the T-helper lymphocytes. Tests such as those on patient DT suggest that Beta can partially correct a defect in T-helper cell function even in the presence of the excessive proportions of T8+ cells observed in AIDS patients.

It appears, further, that some minimal level of residual T-helper cell function must be present for Beta to improve immunological functions; if T-helper cell loss is too severe, there may not be enough T-helper cells left to respond to doses of Beta as a lymphokine and thus be immunologically reconstituted. The data above suggest that when the T-helper cell population falls below approximately 100 cells/ mm³, it may be difficult or impossible to reconstitute immunological function.

### EXAMPLE 16 -- Increase of Immune Response

Amplifiers are prepared and purified as described in Example 5, and are pooled, lyophilized, and redissolved in normal saline or other physiologically acceptable vehicle. An effective dose (e.g., 0.1 ml containing a standard dose of Beta-1.0, which is the amount purified from 4 x 10⁵ leukocytes) is injected intradermally. Increased immune responsiveness is monitored by the patient's reactivity to an antigen to which he is known to be sensitive (e.g., tetanus toxoid), comparing reactivity before and after administering the amplifiers.

Amplifiers are administered either individually, or in combination, depending upon the desired effects. The persistence of the systemic modulation produced by administration of the amplifiers varies from patient to patient, and must therefore be monitored periodically with a suitable sensitivity test, e.g., as described above. Additional doses are administered as required to maintain a desired amplification of immunity based upon the professional judgment of the attending physician.

### EXAMPLE 16A - Chemotherapy

A patient undergoing chemotherapy has a reduced immune system response. The attending physician is concerned that the patient may become subject to opportunistic infections and desires to increase the patient's immune system activity. The patient, an adult male, weighs 70 kgm.

The physician determines a dosage amount of Beta-1.11 or 1.12, TG, or TGG, that in his or her medical judgment is appropriate. (For example, an effective dosage amount of Beta-1.11 or 1.12, as described above, or 60 pg of TG or 80 pg of TGG, every week.) This dosage is injected intradermally or subcutaneously.

The patient's immune function is monitored by weekly blood tests measuring the patient's immune capability. Monthly testing by injection of a recall antigen (such as tetanus toxoid) is also carried out.

The physician monitors the patient's progress and increases or decreases the dosage, as indicated by his or her medical judgment.

### V. Use of Dermal Patch

It has been found advantageous to cause dermal absorption of amplifier material, rather than inject it subcutaneously. Such administration is faster, requires less skill, and is somewhat less annoying to patients. Also, a dose of amplifier material may be administered more slowly by this route, so that there is a more long lasting therapeutic effect. Finally, since oral administration may not be feasible, this route of administration has more possibilities than others for eventual consumer self-medication upon need, as, for example, to immediately counteract the adverse effect of a stress situation on the person's immune system, or to treat a chronic case (such as rheumatoid arthritis, diabetes, or anergy due to age) in the interim between periodic medical monitoring.

### EXAMPLE 17--Patch Application of Amplifiers

A patient suffering from immune system deficiency is treated with Beta-1.0 of preceding Example 5, as follows. The patient's forearm is cleansed with 70% isopropyl alcohol and permitted to dry. An emery board (drugstore type), which has previously been pre-sterilized by autoclaving, is used gently to abrade the patient's skin surface; the skin is stroked 5-6 times with the board. The skin area is recleansed with 70% isopropyl alcohol and permitted to dry.

A medically determined dosage of Beta-1.0 is placed on the gauze portion of a small sterile bandage strip (e.g., Johnson & Johnson "Band-Aid"™), approximately 25 mm x 15 mm, which is then applied over the abraded area.

While a medically determined dosage is a matter of the discretion of the prescribing physician, a standard dose of Beta is believed appropriate.

The same procedure may be used with Eta, Zeta-2, and other amplifier materials.

### VI. Vaccines and Diagnostics

The amplifiers, either singly or in combination, can be used to produce an immune response to weak vaccines. Many pathogens, including several Staphylococcus varieties and fungi responsible for Histoplasmosis or Candidiasis, fail to provoke a strong immune response in certain patients. Moreover, there is no known satisfactory vaccine for conferring immunity on such patients. Such fungal infections are especially dangerous for patients subjected to cancer chemotherapy, or immunosuppressive drugs.

It is possible to vaccinate patients against such pathogens by enhancing their immune response to these weak antigens. This is accomplished by concurrently administering an effective dose of the weak antigen and an effective dose of amplifier. Patients about to receive chemotherapy or transplant surgery can thus be vaccinated prior to treatment, to reduce their susceptibility to histoplasmosis or candidiasis. Used as described below, amplifiers are expected to expand the scope of preventative measures in medicine, and to enlarge the range of weak antigens which can be used for immunization.

### EXAMPLE 18 -- Vaccination

Vaccine is preferably prepared by combining amplifier materials of Examples 4, 5, or 6 with antigens of the desired pathogen, prepared according to known methods in the art to ensure adequate attenuation and sterility. The vaccine is then administered by standard procedures. The subject is thus immunized against the pathogen.

The antigen may be chemically linked to the amplifier, instead of merely mixing the two products together, to assure appropriate receptor site stimulation. The chemical linkage may be by means of a complex, covalent bond, ionic bond, or hydrogen bond.

Since amplifier materials can accelerate the onset of DH reaction to antigen, it is possible to use them to provide a more rapid diagnostic test for determining whether a subject has been exposed to a given pathogen or other antigen. For example, a more rapid test for exposure to tuberculosis is desirable. It presently requires 24 to 36 hours to obtain a TB test result. That length of time requires that the test subject return to the clinic for a second visit, causing expense and inconvenience, or else the subject must mail in a post card (which may be inaccurate or unreliable). The economic advantage of an accelerated diagnostic test is evident.

### EXAMPLE 18A -- Accelerated TB test

Patient A is known to test positive to tuberculin. Patient B is known to test negative.

Preparation C is prepared, consisting of 0.5 tuberculin units of Purified Protein Derivative (Parke Davis Tuberculin-Aplisol, PPD) in 0.2 ml saline. Preparation D is prepared by combining 0.1 ml of Preparation C with 0.1 ml of saline containing approximately 10 femtograms (fg) of Beta-1.12.

Patients A and B are each injected at two sites with 0.1 ml of Preparation C and 0.1 ml of Preparation D, respectively. The following dermal reactions (mm x mm) are observed with respect to Preparations C and D, respectively:

| Hours | Patient A | | Patient B | |
|---|---|---|---|---|
| | C | D | C | D |
| 5 | 0 | 18x20 | 0 | 0 |
| 8 | 0 | 16x18 | 0 | 0 |
| 24 | 18x20 | 16x18 | 0 | 0 |
| 36 | 16x18 | 10x12 | 0 | 0 |

### VII. Efficacy Data on More Highly Purified forms of Amplifier Beta

Additional purification processes were described above, whereby Beta-1.0 is more highly purified and made free from undesired extraneous material, such as phenylalanine, thereby producing Beta-1.1, 1.11, and 1.12. In the DH tests of Examples 7, 7A, and 7B, it was shown that one fg (10⁻¹⁵ g) of Beta-1.11 has approximately the effect of 5-10 fg of Beta-1.0. It may be anticipated, therefore, that the effective therapeutic dosage amounts of Beta-1.1, 1.11, and 1.12 (by weight) are lower than that for Beta-1.0, and also that more repeatable results are obtainable. (However, it should be noted that at this stage of knowledge about Beta-1.1, 1.11, and 1.12, it is not possible to conclude that the materials eliminated from Beta-1.0 to provide Beta-1.1, 1.11, and 1.12 have no function at all in the immunological system.)

The following data suggest the greater intrinsic immunological activity of the more highly purified products. In general, it appears that 1 fg of the molecular species products, Beta-1.11 and 1.12, is approximately equivalent in biological effect to 5-10 fg of Beta-1.0. That is suggested by the DH assay data of Examples 7, 7A, and 7B, and the following data of Examples 19, 20, 21, and 21A. In addition to the DH assay data of Examples 7A and 7B, three types of data have been developed for Beta-1.11 (the Tyr-Gly containing material). They are tests of: (1) in vitro IL-2 receptor expression; (2) in vitro gamma-interferon production; and (3) in vivo clinical data for two ARC patients.

### EXAMPLE 19--Effect of Beta-1.11 on T-helper Cell Receptor Expression

Serial dilutions of Beta-1.11 were prepared, beginning with a 1/1000 dilution and then serially diluting that further by factors of 1, 2, 4 . . . 512. The 1/1000 dilution produces a preparation that is a 46.5 pM concentration of Beta-1.11, while the 1/512,000 dilution produces a preparation that is a 91 fM concentration of Beta-1.11. A tetanus toxoid (TT) preparation was prepared of strength 0.1 flocculation unit (L_{f}) per ml.

Cell cultures were prepared with the TT preparation alone, and with the TT preparation combined with the serial dilutions of Beta-1.11. The cultures were incubated and mixed with a suitable antibody against IL-2 receptors. Data for receptor expression was tabulated both for T-helper cells bearing a low density of receptors and for T-helper cells bearing a high density of receptors. (The T-helper cells bearing a high density of receptors are considered to be the ones that are immunologically active.) The cells were from normal subjects.

In the case of the TT preparation alone, 4.7% of T-helper cells expressed a low density of IL-2 receptors and 0.40% of T-helper cells expressed a high density of IL-2 receptors.

There was a plateau of maximal expression for T-helper cells bearing a low density of receptors from TT + 1/4000 Beta-1.11 (9.9%) to TT + 1/64,000 (8.4%). The highest figure for high density of receptors was at 1/512,000 (9.9%). This highest figure occurred at a point where there was a steadily ascending curve. Accordingly, it is believed that if further dilutions of Beta-1.11 had been used a still higher figure would have been reached, before the percent began to drop. It is not possible to state, from this data, where the maximum would occur, but it seems likely to be somewhere between 1/1,024,00 (45 fM) and 1/4,096,000 (11 fM).

The low density plateau reflected an approximately doubled expression rate of IL-2 receptors as a result of Beta-1.11. The more relevant high density maximum reflected an increase of IL-2 receptors by a factor of approximately 25 as a result of Beta-1.11, and that figure probably falls short of the increase that would result from using still more diluted Beta-1.11.

### EXAMPLE 20--Effect of Beta-1.11 on Antigen-Induced Production of Gamma Interferon

Preparations of 0.1 L_{f}/ml and 1.0 L_{f}/ml TT were used to determine the effect of Beta-1.11 on production of gamma-interferon in normal cells. As before, serial dilutions of Beta-1.11 were prepared from 1/1000 to 1/512,000 (or 46.5 pM to 91 fM).

The 0.1 L_{f}/ml and 1.0 L_{f}/ml TT preparations, alone, respectively induced production of 1.0 and 10.4 units/ml of gamma-interferon.

0.1 L_{f}/ml TT + 1/256,000 Beta-1.11 produced 29.8 units/ml, the maximum, and 0.1 L_{f}/ml TT + 1/512,000 Beta-1.11 produced 29.1 units/ml, so that the optimal dilution of Beta-1.11 here was between 182 fM and 91 fM.

1.0 L_{f}/ml TT + 1/512,000 produced 25.6 units/ml, the maximum.

In the case of the 0.1 L_{f}/ml TT preparation, the maximum was approximately 30 times the baseline amount (between 45 and 91 fM). In the case of the 1.0 L_{f}/ml TT preparation, the maximum was approximately 2.5 times the baseline amount (at approximately 91 fM).

In both TT + Beta-1.11 immunological tests, expression of high-density IL-2 receptors and antigen-induced production of gamma-interferon, the optimal concentration of Beta-1.11 appeared to be approximately 90 fM. These two assays are considered generally indicative of immunological effect, and were found specifically correlated to clinical immunological effect in the case of other Beta materials from which Beta-1.11 is derived. I therefore conclude that the as says indicate a high probability that Beta-1.11 can produce in vivo amplifier effect in human subjects.

I have developed data consistent with such inferences in tests conducted with two ARC patients who were treated with Beta-1.11. The dose used was 0.1 pg (= 100 fg), every two weeks.

### EXAMPLE 21 - Three-month Dosage of Beta-1.11

Patient A received a total of six doses of 0.1 pg of Beta-1.11, given biweekly for approximately three months. His immune responsiveness was measured by determining the reactivity of his lymphocytes to pokeweed mitogen (PWM).

A baseline responsiveness was established before treatment at 6241 units, hereinafter designated as 100%, for comparison purposes. His subsequent PWM reactivity measurements are tabulated below:

| Response after Dose No. | Units | Percent |
|---|---|---|
| 0 (baseline) | 6241 | 100% |
| 1 | 14,027 | 225 |
| 2 | 13,875 | 222 |
| 3 | 12,058 | 193 |
| 4 | 11,753 | 188 |
| 5 | 10,358 | 166 |

It was also observed that A regained his DH reaction to tetanus toxoid as a result of treatment with Beta-1.11.

The trend of observed PWM reactivity suggests that 0.1 pg was an excessive dose, at least for this patient. That is, once the initial doses of 0.1 pg began to reconstitute A's immune system, further doses of 0.1 pg produced a higher than optimal concentration of the material in A's body. The inventor has observed that there is an optimal dosage of amplifier, and that doses above or below the optimum amount produce less amplifier effect than the optimal dose does (see, e.g., '379 patent, col. 13-14, 16-17).

### EXAMPLE 21A - Two-month Dosage of Beta-1.11

Patient B had previously been treated with a series of standard doses of Beta-1.0 to reconstitute his immune system. He was then given biweekly doses of 0.1 pg of Beta-1.11 for a total of four doses.

His immune function was measured by determining PWM reactivity, as in the case of Patient A. His reactivity throughout remained at slightly below normal levels. His DH response to tetanus toxoid also remained at slightly below normal levels.

The data for Patient B suggests that 0.1 pg was approximately the correct dosage amount for this patient (i.e., an effective dosage amount).

Both in vitro and in vivo tests have been made of the synthetically derived immunoamplifier products Tyr-Gly and Tyr-Gly-Gly. The following data is illustrative. The bulk of data obtained by the inventor suggests that the synthetically derived dipeptides and tripeptides are less immunologically active than the naturally derived products Beta-1.11 and 1.12, on a weight basis.

### EXAMPLE 22 - TGG DH test

Purified TGG of Example 6C was dissolved into sterile saline, to a final concentration of 3 x 10⁻¹³ M. That preparation was designated as Preparation A. Further 1:10 successive dilutions were prepared from Preparation A, resulting in Preparations B, C, and D, with the following molar concentrations of TGG:

| A | B | C | D |
|---|---|---|---|
| 3x10⁻¹³ | 3x10⁻¹⁴ | 3x10⁻¹⁵ | 3x10⁻¹⁶. |

Samples of 0.1 ml each were injected intradermally into a recipient test subject, in combination with an antigen to which the recipient had previously been shown to be sensitive. The antigen employed was 0.05 ml of tetanus toxoid (1/20 dilution of standard fluid tetanus toxoid, Squibb/ Connaught). In addition, the same amount of tetanus toxoid antigen was injected in combination with 0.1 ml of normal sterile saline without any TGG, as a control (Preparation E). After 6, 10.5, and 23 hours, respectively, the injection sites were examined and the dimensions of the lesions (mm x mm) were as follows: (The test subject, a normal adult human male, weighed approximately 70 kg.)

| Prep. | Conc. | 6 hrs. | 10.5 hrs. | 23 hrs. |
|---|---|---|---|---|
| A | 3 x 10⁻¹³ M | 12 x 10 | 15 x 17 | 15 x 15 |
| B | 3 x 10⁻¹⁴ M | 14 x 12 | 15 x 18 | 16 x 18 |
| C | 3 x 10⁻¹⁵ M | 17 x 18 | 20 x 22 | 18 x 17 |
| D | 3 x 10⁻¹⁶ M | 12 x 11 | 14 x 14 | 10 x 11 |
| E | saline control | 7 x 7 | 9 x 9 | 13 x 12 |

As an additional control, 0.1 ml of TGG, Preparation C, above, was injected into the test subject without antigen. No reaction was observed over a 48 hour period.

It is seen from the preceding data that 0.1 ml of 3 femtomolar solution (1 femtomole = 1 x 10⁻¹⁵ GMW; 1 femtomolar = 1 x 10⁻¹⁵ M; both are abbreviated hereinafter as 1 fM) at 10.5 hours, which is a total dosage amount of 3 x 10⁻¹⁹ moles, produces the maximum response. Dosages of 0.1 ml of 30 fM and 300 fM solutions produce less effect than 0.1 ml of 3 fM solution, because increasing the dosage beyond the optimum produces a paradoxical effect, viz., lessened immune reaction with creasing amplifier dosage. Similar effects are noted in the inventor's '379 patent. It is also seen from this data that decreasing the dosage from 3 fM to 0.3 fM produces less lesion, since 3 fM is approximately optimum for maximum immune effect in this test.

The great sensitivity of the human immune system to TGG is shown by these tests. A dose of 0.1 ml of 3 fM solution of a therapeutic agent is a very minute dose (0.0003 femtomoles). One femtomole of TGG is approximately 277 x 10⁻⁹ micrograms. A quantity of one picomole (1 pM = 1 x 10⁻¹² GMW) of TGG is approximately 277 x 10⁻⁶ micrograms.

### EXAMPLE 23 - IL-2 in vitro test

Tests were carried out to determine the ability of TGG to stimulate in vitro production of IL-2 in response to mitogen stimulation, in normal subjects. The mitogen PHA (Wellcome Reagents HA 16) was used, with and without TGG added to the sample. The production of IL-2 in response to mitogen was characterized by the existence of the same optimal concentration phenomenon (paradoxical effect) described above. Data was collected to show the ratio of PHA-stimulated IL-2 production with TGG added to PHA-stimulated IL-2 production without TGG added.

In subject #1, the ratio at optimal concentration of TGG was 1.45. In subject #2, the ratio at optimal concentration of TGG was 2.55. It is believed that this data shows a significant effect of TGG on mitogen-stimulated IL-2 production.

### IX. Discussion of Preceding Data

On the basis of the foregoing data, it is possible to characterize Beta-1.0 and the other amplifier materials of this invention more precisely. It is also possible to describe more precisely the procedures for purifying them and separating them from one another and from other materials.

### A. HPLC/O.S. Elutability Data

These amplifier materials are all characterized by being O.S. elutable with an acetonitrile-in-phosphate gradient of appropriate pH. (As used hereinafter, "O.S. elutable" with an acetonitrile-in-phosphate gradient means: capable of being eluted from octadecyl silane by means of reverse-phase high pressure liquid chromatography with an acetonitrile-in-phosphate gradient of increasing acetonitrile concentration. This definition is similar to that found in col. 15 of the '379 patent.)

The property or parameter of being O.S. elutable under certain conditions permits the further characterization and separation of these amplifier materials because they can be ordered in terms of their relative elutability. Beta-1.0, for example, is O.S. elutable in the portion of the gradient containing only 0.4-1.5% acetonitrile in the effluent. Amplifier Zeta-2 is O.S. elutable only in a much greater concentration of acetonitrile, and so on. As discussed below, this fact provides information about the chemical constituents of the molecule. Moreover, it appears that to effectuate the same elution in a more acid solution a higher acetonitrile concentration is required, which suggests some guidelines for future trial and error development of additional HPLC gradient systems.

Each of these amplifier materials can therefore be characterized in terms of several parameters:
(1) the given amplifier material is primarily O.S. elutable in a specific zone of the gradient, from lower acetonitrile concentration limit a to upper concentration limit b;
(2) The amplifier material is primarily not O.S. elutable in the zone of the gradient below a;
(3) the amplifier, when properly purified from extraneous material, is substantially free of material that is O.S. elutable in the zone below a;
(4) the amplifier, when properly purified from extraneous material, is substantially free of material that is O.S. elutable primarily in the zone above b; and
(5) for any given amplifier material there may be an inverse correlation between gradient pH and acetonitrile concentration defining the elution zone of the gradient.

The third and fourth items are explained in the '379 patent (at col. 15), and said discussion is incorporated herein.

As indicated earlier, following Example 3, one must distinguish between input solvent concentration for a gradient and output or effluent solvent concentration. The latter is always somewhat lower, at a given moment. Since the former is not an invarient, input concentration figures must always be stated very approximately, and depend on the other operating parameters. Input concentration figures are also a matter of choice, to some extent. That is, in order to assure that there is an effluent concentration zone including percentages a through b it is necessary, at least in principle, only to start the gradient below a and end it above b. But when the gradient ends just barely above b, it can take an indefinitely long time (because of a lag effect) for the effluent solvent concentration to reach b. Accordingly, a person skilled in this art will select a gradient ending sufficiently beyond b to accomplish the separation in a reasonable time and at a reasonable labor cost.

Some information about the nature of the molecule is provided by the HPLC data. In reverse phase HPLC, non-ionic compounds elute in reversed order of polarity. The most polar compound elutes first. Thus, Beta should be more polar than Zeta-2 since Beta elutes first. Generally speaking, a molecule X to which is added a hydrocarbon or hydrophobic group, such as alkyl or benzyl, will be less polar than the same molecule X to which is added a hydrophilic group, such as an NH₂ or COOH group. Thus, Beta is likely to have a more hydrophilic component than Zeta-2; Zeta-2 is more likely to have a relatively greater hydrocarbon composition than Beta (or else more carbon-carbon double bonds, or both).

The Beta 1.11 material associated with a Tyr-Gly dipeptide material elutes after the Beta 1.12 material associated with a Tyr-Gly-Gly tripeptide material. This appears to be true for both the natural materials and the synthetically derived chemicals. This represents anomalous elution behavior since, in general, smaller molecules bind less tightly to O.S. columns and are therefore likely to elute earlier than larger molecules. However, relative hydrophobicity also plays a role. The Tyr group has a benzene ring, which tends to be hydrophobic, and Tyr constitutes a relatively larger portion of Tyr-Gly than it does of Tyr-Gly-Gly.

### B. Ultraviolet absorption data

Furthermore, any given amplifier can be characterized in terms of the ultraviolet absorption profile (hereinafter "UVAP") associated with the HPLC system and the position of the amplifier in that profile (hereinafter "UVPP," ultraviolet profile position). UV absorption is known to be associated with particular types of molecular bonds. For example, absorption at 210 nm and 285 nm are each associated with peptide bonds, among others; and absorption at 254 nm is associated with the presence of a carbon ring group, among other things. Hence, UVAP data provides both a repeatable "fingerprint" for, or a "pointer" to, elution of a molecule whose structure may be unknown, and UVAP data also indicates the possible chemical groups present in a molecule.

I have found the UVAP and UVVP to be good indicators in the processes described hereinabove, and as a practical matter they have been quite satisfactory as as tools that permitted me to make purifications that repeatably yielded pharmaceutically useful products. It is believed that UVAP and UVPP are coming to be recognized by those skilled in this art as a useful means of characterizing biologically active substances that are difficult to refine or purify down to the molecular level. In this regard, UVAP/UVPP is a parameter of the physical and chemical nature of the product analogous to its M.W., solubility in particular solvents, smell, taste, and other parameters short of a molecular diagram of the structure of the product; and it is highly appropriate to use UVAP/UVPP to identify and characterize a product in a way that can distinguish it from other products.

Figures 1 to 3 will assist those of skill in the field to recognize where particular materials will elute, despite variations in HPLC parameters, in that the figures provide at the very least an "I know it when I see it" criterion. In the discussion that follows, a more precise characterization of the amplifier materials of this invention in terms of the UVAP is set forth. In the final portion of this specification, immediately preceding the claims, the terms "UVAP" and "UVPP" are defined more specifically as they are used in the claims.

### C. Amino acid sequence data

Finally, at least insofar as the various Beta materials are concerned, there is amino acid sequencing data indicating that the only amino acid residues present in Beta in substantial amounts and associated with intrinsic amplifier activity are Tyr and Gly. Hence, the active components of Beta may properly be considered the dipeptide Tyr-Gly and the tripeptide Tyr-Gly-Gly, or derivatives thereof. (By "derivative" is meant a methylated, acetylated, hydroxylated, etc. form of the molecule, or an ester, or another molecule derived from the stated molecule. The concept is particularized in more detail below.)

### D. Description of amplifiers

The foregoing descriptive and characterization data for the amplifier materials of this specification can be summarized and explained as follows:

### Beta

Beta has both an accelerating and augmenting effect on the DH skin response of recipients sensitive to a given antigen. See Example 7. The reactions produced by Beta administered with antigen reach peak intensity at about 6 to 24 hours after intradermal injection and fade rapidly thereafter. (In contrast, normal DH response, in the absence of amplifier, reaches a peak 24 to 30 hours after injection of antigen.) Maximal amplifying activity is observable at an optimum concentration, with greater or lesser concentrations than the optimum giving a reduced amplification of DH response.

Beta is O.S. elutable between approximately 0.4% to 1.5% (v/v) acetonitrile concentration. That is in the portion of the pH 5 acetonitrile-in-phosphate HPLC gradient where the effluent has a refractive index of from approximately 1.330 to 1.333. Beta is characterizable in terms of its ultraviolet absorption profile (UVAP) in the pH 5 process as a material that is associated with a distinct peak following a doublet peak (Alpha) and preceding a distinct broad lower peak (Gamma) or a shoulder (also Gamma) on its own peak. Gamma is followed by an interval (trough) without substantial ultraviolet absorption.

The intrinsically active component or components in Beta pass through a dialysis membrane having a nominal M.W. cutoff of 3500. In this relatively less purified form, Beta may be the same as, or a moiety of, Amplifier 1 of the '379 patent.

The principal active component of Beta has been designated herein as Beta-1.11. This product appears to be the dipeptide Tyr-Gly, which has M.W. 239. Another component found in Beta is Beta-1.12, which has been identified with the tripeptide Tyr-Gly-Gly, which has M.W. 295. As stated above, however, Beta-1.11 and 1.12 may be complexed with one another and/or ions such as Zn++, so that they cannot be considered necessarily to be the dipeptide and tripeptides, per se. The possibility of isomers must be considered, as well, and so too must addition of other groups (e.g., methyl, acetyl, amide). However, it can be concluded that the products consist "essentially" of Tyr-Gly and Tyr-Gly-Gly, whether in that form alone or in association with other groups.

Beta has been purified as described above and then subjected to the ethanol-water HPLC process of the '379 patent. The results show elution of Beta-1.1 in the same region of the gradient as Amplifier 1 of the '379 patent. This suggests, but does not establish, that Beta-1.1 is Amplifier 1 or a moiety thereof. It is also possible that Beta-1. Given the present state of information, it is not possible to determine the exact relationship between Amplifier 1 and the Beta moieties. It may be that Beta-1.1 represents part of Amplifier 1, while other amplifiers described herein, such as Zeta, are other parts of Amplifier 1.

### Delta

Delta causes augmented and prolonged response to antigen. See Example 8. Delta's biological activity is generally similar to Beta's.

Delta elutes from the pH 5 gradient where the acetonitrile concentration is approximately 2.8 to 3.3%. Delta is characterizable in terms of its UVAP in the pH 5 process as the first distinct peak in a group of three peaks following the space (trough) in the absorption profile after the Gamma peak or shoulder. However, Delta is not always recovered separately in the pH 5 process, particularly if the pH deviates from 5.0. Presumably the molecules in Delta are less polar in structure than those of Beta, since the latter elute first.

Delta is not recoverable from the 2.5 pH trifluoracetic acid cleanup process of Example 5. It may have bonds susceptible to acid hydrolysis.

### Zeta

Zeta also causes both an accelerated and augmented response to antigen, and is generally similar to Beta in activity, although the degree of acceleration is somewhat less rapid than the response to Beta. Maximal amplifying activity with Zeta, as with Beta, is observable only at an optimum concentration, with greater than optimal concentrations giving reduced amplification or even suppression of the DH response.

Zeta is O.S. elutable in the portions of the pH 5 acetonitrile-in-phosphate gradient between approximately 3.5 to 4.1%, where the refractive index of the effluent is from approximately 1.344 to 1.345. Zeta is characterizable in terms of its UVAP in the pH 5 process as the last of a distinct group of three peaks (two, if Delta fails to come off) following the space (trough) in the absorption profile after the Gamma peak or shoulder, and preceding either a shoulder (Eta) on a distinct broad peak (Theta) or else preceding a small peak (Eta) followed by a distinct broad peak (Theta). In the cleanup process of Example 5, Zeta-2 is the third major peak.

Presumably the molecules in Zeta-2 are less polar in structure than those of Delta, since the latter elute first. By the same token, Zeta-2 is less polar than Beta (or than Tyr-Gly or Tyr-Gly-Gly). As indicated above, Zeta may be a component of the amplifier previously identified as Amplifier 1.

### Eta

Eta also causes both an accelerated and augmented response to antigen, and is generally similar to Beta, although the degree of acceleration appears to be somewhat less rapid than the response to Beta.

Eta is O.S. elutable in the portions of the pH 5 acetonitrile-in-phosphate gradient between approximately 3.7 to 4.4%, where the refractive index of the effluent is from approximately 1.347 to 1.353. Eta is characterizable in terms of its UVAP in the pH 5 process as following the last (Zeta) of a distinct group of three (two, if Delta fails to come off) peaks following the space (trough) in the absorption profile after the Gamma peak or shoulder, and as being a small broad peak or a shoulder preceding (or included in) a distinct broad peak (Theta).

Presumably the molecules in Eta-1, the more highly purified form of Eta, are less polar in structure than those of Zeta-2, since the latter elute first.

### Pi

Pi also causes both an accelerated and augmented response to antigen, generally similar to Beta. Pi is O.S. elutable in the portions of the pH 2.5 acetonitrile-in-phosphate gradient between approximately 12.0 to 13.5%. Pi is characterizable in terms of its UVAP in the pH 2.5 process as a doublet peak in the fifth position among nine peaks. Typically, Pi is preceded by a higher peak (Omicron) and followed by two close, higher peaks (Rho and Sigma). The Omicron peak is preceded by a deep trough.

### Sigma

Sigma also causes both an accelerated and augmented response to antigen, generally similar to Beta, although the degree of acceleration appears to be somewhat less rapid than the response to Beta. Sigma is O.S. elutable in the portions of the pH 2.5 acetonitrile-in-phosphate gradient between approximately 14.0 to 14.5%. Sigma is characterizable in terms of its UVAP in the pH 2.5 process as the seventh of nine peaks, typically preceded very closely by a higher peak (Rho) and followed by a lower peak or trailing shoulder (Tau) and then a distinct high peak (Upsilon). It is possible that Sigma of the pH 2.5 process may be the same material as Beta of the pH 5 process.

Presumably the molecules in Sigma are less polar in structure than those of Pi, since the latter elute first.

### Upsilon

Upsilon causes an augmented DH response and is generally similar to Beta in activity. See Example 9. Upsilon elutes at approximately 15.8 to 16.0 acetonitrile concentration in the pH 2.5 process.

Upsilon is characterizable in terms of its UVAP in the pH 2.5 process as the ninth of nine peaks, following a descending profile trailing the seventh peak (Sigma); the descending profile is interrupted by a lesser peak or a shoulder (Tau). It is possible that Upsilon of the pH 2.5 process may be the same material as Delta of the pH 5 process.

Presumably the molecules in Upsilon are less polar in structure than those of Sigma, since the latter elute first.

### GENERAL CONCLUDING REMARKS

The above described amplifiers of the immune system regulate the immune response, directly with respect to cell mediated immunity and perhaps indirectly affecting humoral immunity as well. The materials have been prepared with a high degree of purity such that their properties have now been characterized and shown to be entirely and unexpectedly different from transfer factor and from partial fractionations of transfer factor reported in the prior art. Furthermore, the materials of this invention have been purified sufficiently to permit their administration to human subjects to produce beneficial effects, which has produced beneficial results without known harmful side effects.

Some of the materials disclosed and claimed herein are defined largely in terms of their biological activities and physical properties, because that is the type of data that exists at this time. The materials do not necessarily consist of single molecules or chemical entities, although their discovery has led to the discovery of a dipeptide and a tripeptide chemical (Tyr-Gly and Tyr-Gly-Gly, respectively) molecule having intrinsic amplifier activity in vitro and in vivo. Some of the constituents present in partially purified products lack intrinsic amplifier activity, but they may play some role in facilitating the amplifier activity of the components shown to have intrinsic activity.

### Products

The amplifier materials herein described are medically useful for the treatment of patients suffering from a variety of hypoimmune conditions. Among other conditions, AIDS and ARC appear to respond favorably to amplifier treatment. A large number of clinical tests on individuals suffering from AIDS and ARC show significant clinical improvement as a result of administration of Beta moieties. Furthermore, there have been no reports of toxic effects from administration of these amplifier products, so that they appear particularly promising for therapeutic use.

It is especially significant that these materials may be isolated from normal individuals, rather than from specific identified donors, so that large-scale purification from pooled sources is feasible. Moreover, the invention includes the discovery that some amplifier material contains low-M.W. peptide products as intrinsically active components. That discovery opens the way to basically chemical, rather than basically biological, preparation of amplifier compositions. As described in detail below, that discovery also paves the way to molecular manipulation of the products already discovered, in order to improve their pharmaceutical properties. It is also considered, therefore, that the invention includes the products of molecular manipulation suggested hereinafter.

The invention is considered to include the therapeutic use of the products, and also the pharmaceutical compositions in which the products are the active ingredients.

### HPLC processes

The invention is also considered to include the novel processes for purification and extraction of these new materials, described herein. The development of several HPLC acetonitrile processes has provided some general principles for determining the identity of other similar systems. Accordingly, the scope of the invention includes other HPLC systems in conformity with, or using the teachings of, such principles. The principles are summarized as follows:
First, it is not critical what the exact shape of the gradient is. Rather, it is important to determine where in the gradient the materials of interest come off the column. That part of the gradient should not be crowded, so that dc/dt, where c represents solvent concentration, should have a relatively low value at the points where materials of interest come off (and preceding such points, because of the lag effect previously mentioned). Therefore, the gradient curve should be relatively flat at and before points where useful material comes off or where a separation of materials is desired. If a single gradient curve meets this requirement, it may be selected. Otherwise, a series of curves should be joined (as in Examples 4 and 6).

Also, as previously indicated, when an input gradient is specified in terms of solvent concentrations, the range is approximate because of the lag factor, and the appropriate range will therefore vary depending on both other operating parameters and how long the user wants to permit the process to take. (For example, in the procedure of Example 6A, a 0% to 50% gradient was used to elute Beta-1.0 material that comes off the column in an effluent with a solvent concentration of approximately 0.1% to 0.4%. The gradient could instead have been programmed from 0% to 25% or 10% or 5%, and eventually the 0.4% point would be reached, given enough time.)

In determining how to use a gradient, the parameters of retention time and UVAP (discussed below) are helpful in realizing reproducible results. Because retention time shifts with the age of a column, it may be useful to calibrate it with a known marker, as is explained above in the Beta-1.11 process where purified commercial Tyr-Gly-Gly can be used as a marker.

Second, the pH must be adjusted to provide a good yield for the solvent system used. As previously indicated, there appears to be an inverse correlation of pH and acetonitrile concentration for effective separation in this process. The precise pH adjustment is a matter of trial and error.

Third, phosphate buffer has been found effective for this procedure, at 0.02 M. If a different concentration of buffer is used, or a different acidic ion such as acetate or sulfate, then the solvent concentrations and the proper adjustment of pH will be different. Compare Examples 4, 5, and 6, which illustrate this.

Fourth, a relatively basic buffer (see Example 4) leaves ionic contaminants and extraneous material in the fractions of interest. The former may have to be removed (see Example 5). On the other hand, a relatively acidic buffer may destroy some materials of interest (see Example 5). Thus, there is a tradeoff.

Finally, in evaluating a particular gradient, the ultraviolet absorption profile (UVAP) is very helpful in determining whether a good separation is being achieved. (This point is described in further detail below, in connection with determining the identity of an effluent by means of its UVAP position.

While the invention has been described in connection with specific and preferred embodiments thereof, it will be understood that it is capable of further modifications without departing from the spirit and scope of the invention. This application is intended to cover all variations, uses, or adaptations of the invention, following, in general, the principles of the invention and including such departures from the present disclosure as come within known or customary practice within the art to which the invention pertains.

### DISCUSSION OF TERMINOLOGY OF CLAIMS PRESENTED FOR EXAMINATION

The claims presented hereinafter utilize the terminolgy employed hereinbefore. The lexicography, insofar as it differs at all from ordinary usage is based on the preceding disclosure and should be understood in the light thereof. The following discussion further particularizes some aspects of the terminology and lexicography used in the claims.

The term "**pH-adjusted**" refers to alteration of pH with base, acid, or buffer, such as in Examples 4, 5, and 6. The term "**aqueous-phosphate-solution**" refers to an alkali or alkaline earth (strong base such as K, Na, Ca, Mg, etc.) phosphate buffer or solution, such as that described in Examples 4 and 6.

Concentration percentages for gradients are expressed on a (v/v) basis. When the word "**approximate**" is used to describe the input concentration of a solvent in a gradient, the figure stated is based on considerations described above, which make the concentration used to a considerable extent a matter of operator choice. As indicated above, the upper limit can in principle be reduced to almost the upper limit of desired effluent concentration, but this will occur at the expense of time and therefore increased labor costs and lower throughput in a given time. Such expedients will be inconvenient, but will work, albeit not in what the inventor considers the best mode of practicing the invention. Hence, such expedients should be considered within the spirit and scope of the invention, and within the concept of the word "approximately" (or else as constituting an equivalent to the stated percentages). It should be noted that in product claims the input gradient's solvent concentration figures are stated in relation to an input gradient that can elute the desired product. Therefore the stated concentration limit does not exclude the possibility (indeed, it is a fact) that the same product can also be eluted with a somewhat narrower or broader input gradient range.

The term "**extraneous material**" refers to material not having the amplifier activity to which the invention is directed, or at least not having intrinsic amplifier activity.

The term "**UVPP**" ("**ultraviolet profile position**") is used to characterize amplifier materials in terms of where they come off the HPLC column, in relation to other materials coming off the column, and using the shape of the **ultraviolet absorption profile** (**UVAP**, as defined above) as the frame of reference. Thus, to give a hypothetical example, if the UVAP of a process resembled the profile of a human face, one could define a particular material as that material which eluted when the ultraviolet absorption reading for the effluent corresponded to the tip of the nose on the hypothetical ultraviolet absorption profile (UVAP). The UVPP of that particular material would be: "at the tip of the nose," meaning that the material is associated with the tip of the nose part of the hypothetical UVAP.

The UVPP of the hypothetical material would remain recognizable, even though the profile were modified by an increase or decrease in flow rate or by a change in gradient curve, as is shown for actual materials of this invention by Figures 1A through 1C. To those skilled in the art, the topological characteristics of the profile would make it recognizable despite the distortions introduced by modifying the gradient, and the tip of the nose position could still be located.

Hence, the term "UVPP" as used in the claims refers to the topological characteristics (and to some extent also the relative magnitudes of ultraviolet absorption peaks) of the ultraviolet absorption profile of the effluent passing the ultraviolet absorption detector of the machine, in the course of an HPLC procedure wherein solvent concentration monotonically increases with time. The characteristics are expressed relative to the characteristics of materials eluting nearby in the gradient in the course of the procedure. UVPP must be stated with reference to a specific solvent system gradient, such as an acetonitrile-in-aqueous-phosphate-solution of a particular pH, and also should be stated with reference to a particular UV wave length or frequency, such as 210 nm. (Most data used herein is with reference to 210 nm, because that wave length is typically associated with a known peptide bond.)

UVPP data has been provided above in the "Discussion of Preceding Data" section of the specification, in the descriptions of the individual amplifier materials. As noted previously, material passes the ultraviolet absorption detector slightly before leaving the column (e.g., 48 seconds earlier for a 1 ml/min flow rate and a 0.8 ml volume of post-detector tubing) in the equipment described hereinabove.

The term "**O.S. elutable**" is explained in detail in the part of the specification following Example 18. The phrases "primarily O.S. elutable in" and "largely free from material O.S. elutable in" specified zones of a gradient are used at times. That a material is "**primarily O.S. elutable in**" a particular zone of a gradient means that at least 51% of the material in question is O.S. elutable in the specified gradient zone. That a material is "**largely free from material O.S. elutable in**" a particular zone of a gradient means that less than 10% of the material in question consists of material O.S. elutable in the particular zone.

The term "**starter amplifier preparation**" refers to partially purified amplifier or modulator material, such as that described in Examples 1-4. The term "**ACTF 0.1% gradient**" means an acetonitrile-in-trifluoroacetic-acid-solution gradient wherein said trifluoroacetic acid is approximately 0.1% (v/v), the pH thereof is adjusted to approximately 2.5, and the input acetonitrile concentration for said gradient includes the range from approximately slightly above 0% to approximately 25%. The term "**ACTF 0.05% gradient**" means an acetonitrile-in-trifluoroacetic-acid-solution gradient wherein said trifluoroacetic acid is approximately 0.05% (v/v), the pH thereof is adjusted to approximately 2.5, and the input acetonitrile concentration for said gradient includes the range from approximately slightly above 10% to approximately 30%.

References are made in the claims to **polypeptide** molecular species and to **polypeptide**, **tripeptide**, and **dipeptide materials**. As these terms are used in the claims, they refer to molecules that contain a plurality of amino acid residues (i.e., polypeptide, tripeptide, or dipeptide amino acid residue sequences, respectively) but other groups or moieties may be chemically joined or linked thereto. The linkage may be by complex, covalent bond, ionic bond, or hydrogen bond. Such other groups may include, by way of example and without limitation, methyl, ethyl, acetyl, hydroxyl, COOH, amide, pentose, and metal ions such as Zn++, Ca++, Mn++, and Mg++; esters may also be present. For example, when reference is made to molecular species that are **polypeptide materials** the latter term could include all of the following molecular species (some hypothetical), among others: (1) Tyr-Gly, (2) Tyr-Gly-COOC₂H₅, (3) CH₃-Tyr-Gly, (4) Tyr-Gly-Gly, (5) HOOC-Tyr-Gly-Gly, (6) Tyr-D-Ala-Gly, (7) Tyr-Gly-D-Ala-HCl, (8) Gly-Tyr-Gly-Ile, 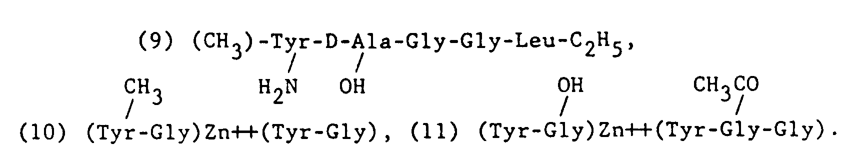
(It will be appreciated, of course, that all of the groups present, including those nonpeptides chemically joined to peptide groups, must be pharmaceutically or biologically acceptable.)

Of the listed examples of molecular species, each is a **polypeptide material**, as that term is used in the claims, and so too is any mixture or complex of two or more of them. Examples (1) to (3) are **dipeptide materials**, and so too is a mixture of them. Examples (4) to (7) are each **tripeptide materials**, and so too is a mixture of them. Example (10) is a dipeptide material complexed to another dipeptide material; example (11) is a dipeptide material complexed with a tripeptide material.

As used in the claims, a dipeptide material complexed with another material, such as another dipeptide material, a tripeptide material, or a nonpeptide, is still a dipeptide material. Similarly, a tripeptide material complexed with another material, such as a dipeptide material, a tripeptide material, or a nonpeptide, is still a tripeptide material. Hence, a reference to a product wherein a particular constituent is a dipeptide material would include those products where the constituent in question is a dipeptide material complexed with some material.

When peptide sequences are enumerated, the usual convention of starting from the N-terminal end is used. The other end (last stated residue) is the C-terminal end. For example, in example (9), a methyl group is at the N-terminal end (where Tyr is) and an ethyl group is at the C-terminal end (where Leu is). Examples (3) and (9) are N-methylated materials, i.e., an N-methylated Tyr-Gly material and an N-methylated pentapeptide material, respectively. Example (2) is an esterified Tyr-Gly material, esterified with an ethyl group at the C-terminal end. (The COO belongs to the Gly amino acid residue and is from the C-terminal COOH normally found at the end of the last amino acid residue of a polypeptide.)

Examples (1) to (5), (7), and (8) contain Tyr-Gly amino acid residue sequences; examples (6) and (9) contain Tyr-Gly amino acid residue sequences into which a D-aminoacid residue has been inserted. Examples (4) and (5) contain Tyr-Gly-Gly amino acid residue sequences; example (9) contains a Tyr-Gly-Gly amino acid residue sequence into which a D-aminoacid residue has been inserted.

It is anticipated that synthetic versions of the naturally occurring polypeptide materials described herein will be developed on the basis of further analytic work that is built on the information disclosed herein, and that such synthetic materials, if purified from unacceptable contaminants, may be used in place of the natural materials derived from human leukocyte extracts. For example, it is known that absorption rate and blood level of pharmaceuticals can be improved by molecular modifications of a natural or earlier-discovered form of a product, and the historical literature contains numerous instances. Thus, chlortetracycline was deschlorinated to provide an improved tetracycline product; a bond in clorothiazide was saturated to produce hydrochlorothiazide, thereby lowering the effective dosage amount by a factor of ten; and numerous synthetic forms of penicillin were provided by modifying the side chain attached to the beta lactam ring, such as by placing a benzyl at its end or replacing an H attached to the side chain with a CH₃ or NH₂, thereby making the product harder to hydrolyze (by action of penicillinase) and otherwise providing desired characteristics not present in the original molecule.

In this connection, the paper of Schwartz, Malfroy, and Baume, op. cit. supra, summarizing various papers on inhibition of enzymatic cleavage of polypeptides, should be considered. They summarize various papers that teach the addition of appropriate moieties to inhibit hydrolysis and inactivation of products containing the peptide sequence H₂N-Tyr-Gly-Gly-...-COOH. These papers suggest N-methylating the Tyr residue to inhibit aminopeptidase hydrolysis and cleavage of the Tyr-Gly amide bond. They also suggest amidification, esterification, and replacement with an alcohol of the C-terminal COOH. In addition, they suggest placing a D-aminoacid residue at or toward the C-terminal end or inserting such a group (such as D-Ala) between the Tyr and Gly residues. Whether these expedients would actually work to inhibit enzymatic action and thus improve the in vivo potency of Tyr-Gly and Tyr-Gly-Gly as immunoamplifiers is uncertain, given the unpredictability of this art. However, it is believed that a skilled person would investigate these promising avenues, so that it is within the teaching of this patent to attempt these expedients.

Such further synthetic products or derivatives are considered to be within the scope of this disclosure and the invention claimed herein. Accordingly, a phrase in the claims such as "tripeptide material wherein the amino acid residue sequence is Tyr-Gly-Gly" is intended to include derivative forms, such as synthetic derivative forms, of Tyr-Gly-Gly. Such derivatives include those described in the preceding paragraph. E.g., derivatives of Tyr-Gly-Gly in which replacements such as CH₃ or NH₂ for H, or an alcohol for a COOH, are made; esters; and extensions of the amino acid residue sequence by insertion of a D-aminoacid residue, such as D-Ala, thereinto. They also include other derivatives known in pharmaceutical art, particularly peptide pharmaceutical art.

In accordance with the foregoing teachings, the term "**inhibitor**," as used in the claims in an phrase such as "to which is bound an inhibitor," means a group such as, by way of illustration, a methyl used for N-methylation of a Tyr residue or an amide used to amidify a terminal carboxyl, as suggested by Schwartz et al. More generally, an inhibitor means any group that can be bound to (or inserted into) a polypeptide material at a site where its presence will inhibit enzymatic cleavage of the polypeptide. As taught by Schwartz et al., esterification of the terminal carboxyl also inhibits enzymatic cleavage and so does replacement of that carboxyl with an alcohol; further, they teach that the insertion of a D-aminoacid inhibits enzymatic cleavage. Accordingly, each of these examples illustrates binding an inhibitor to a polypeptide material in order to inhibit enzymatic cleavage. In the same context, "bound to" is intended to include "inserted into. The foregoing examples of inhibitors are intended to be illustrative, rather than exhaustive.

Further, an inhibitor can be mixed with a polypeptide to inhibit enzymatic cleavage, instead of binding it to the polypeptide. Schwartz et al. describe various inhibitors that are known to inhibit enzymatic cleavage when they are mixed with polypeptides. E.g., bacitracin, puromycin, bestatin, amastatin, and thiorphan. However, the inventor at this time believes that binding is a preferred embodiment to mixing for in vivo use, because of the dilution effect that occurs upon in vivo administration. Hence, while an inhibitor can be included in a pharmaceutical composition for this purpose, simply by admixing it, it is considered preferable to bind an inhibitor to the active molecule in the composition.

It is also possible to place Tyr-Gly or Tyr-Gly-Gly in the bloodstream or at a site where it can act as a lymphokine, by administering to the animal or human subject a related molecule that metabolizes to Tyr-Gly in the body. Schwartz et al. describe how a polypeptide may be enzymatically cleaved to moieties, such as Tyr-Gly and Tyr-Gly-Gly, that are "inactive" or otherwise "useless" (for the purposes of Schwartz et al. and those whose work they describe, who are concerned with opiode peptide effects, and not the effects described herein). They describe how injection of Leu- and Met-enkephalin into rats and mice has resulted in the production of, among other things, Tyr-Gly and Tyr-Gly-Gly metabolites. They suggest that purified angiotensin-converting enzyme (hereinafter referred to as ACE) and dipeptidylcarboxypeptidase (hereinafter referred to as DPCP) can cleave a Gly-Phe amide bond in these polypeptides, producing a Tyr-Gly-Gly moiety; and that the Gly-Gly amide bond may be cleaved by dipeptidylaminopeptidase, producing a Tyr-Gly moiety. They also identify ACE as a form of DPCP, and finally conclude that an enzyme designated as "enkephalin-DPCP" or "enkephalinase" is the principal enzyme that metabolizes the polypeptides of interest to them into "inactive" moieties such as Tyr-Gly and Tyr-Gly-Gly. Of course, the "inactive" moieties of no interest to those workers are the products of interest in the instant invention.

These facts suggest that Tyr-Gly-A-B-C or Tyr-Gly-Gly-D-E-F (where A-B-C and D-E-F are other groups such as Phe-Met or Phe-Leu) can be administered to a human, mouse, rat, or other mammal to produce Tyr-Gly or Tyr-Gly-Gly in the body (once the A-B-C or D-E-F is enzymatically cleaved off to leave the moieties of immunological interest). Since this is a known pharmaceutical expedient, it is considered within the scope of the invention. However, it is not at this time considered a preferred embodiment so much as a possible expedient to attempt to avoid the scope of claims directed solely to Tyr-Gly and Tyr-Gly-Gly, while at the same time appropriating the gist of the invention and the inventor's teachings.

The term "**small peptide amplifiers**" is used in certain claims for pharmaceutical compositions. That term is intended to mean members of the group consisting of Beta-1.0, Beta-1.1, Beta-1.11, Beta-1.12, and Zeta-2, and complexes thereof with one another. Components thereof with intrinsic biological activity have been identified, as described hereinabove, as a dipeptide (Tyr-Gly) material and a tripeptide (Tyr-Gly-Gly) material, in the case of the Beta products. The term is also intended to include synthetic modifications of the foregoing intrinsically active amino acid residue sequences, as described above in defining the terms dipeptide, tripeptide, and polypeptide materials. For example, N-methylated Tyr-Gly would be included as a small peptide amplifier, as would Tyr-Gly in which the terminal COOH group of the Gly moiety was either esterified with or replaced by a pharmaceutically acceptable alcohol such as C₂H₅OH.

In the process claims for processes whereby, for example, Beta-1.1 or 1.11 are separated from extraneous material by means of an ethanol/water and/or an acetonitrile/trifluoroacetic acid system, the claim is written in the "Jepson" format, thereby improving clarity and permitting brevity in the description of what is claimed. Use of that format is not intended to imply that the subject matter in the preamble of the claim was old at the time of the invention.

## Claims

1. A composition of matter that is characterized by the properties that it:
(a) increases the speed, magnitude, or duration of a response of the immune system of a human body, where said response:
(1) is to the introduction to said body of at least one antigen to which said body has been previously or is concurrently exposed,
(2) is specifically attributable to a function of the human immune system, and
(3) occurs following said introduction of said antigen;
(b) is substantially free of other, different material that has an effect on said immune system response opposite to the effect of said composition of matter on said response;
(c) is substantially free of substances that transfer an antigen-specific immune system response to an antigen to which said body has not been previously exposed and is not concurrently exposed;
(d) consists essentially of polypeptide materials having an average molecular weight greater than approximately 200 and lower than approximately 1500;
(e) is O.S. elutable with an acetonitrile-in-aqueous-phosphate-solution gradient; and
(f) is substantially free from pharmaceutically unacceptable constituents.

2. A composition of claim 1, wherein the polypeptide material has molecules containing no more than six peptide groups.

3. A composition of claim 2 wherein the polypeptide material is a dipeptide containing Tyr-Gly as the only amino acid residue sequence.

4. A composition of claim 2 wherein the polypeptide material is tripeptide containing Tyr-Gly-Gly as the only amino acid residue sequence.

5. A composition of claim 3 or claim 4 wherein the Tyr residue is N-methylated.

6. A composition of claim 3 or claim 4 wherein the carboxyl group at the C-terminal end of the amino acid residue sequence is amidified or esterified.

7. A composition of claim 2 containing in the polypeptide material a D-aminoacid residue at a position other than at the N-terminal end of the amino acid residue sequence.

8. A composition of claim 2 to which is bound an inhibitor.

9. A composition of claim 2 wherein at least two polypeptide materials are complexed; each said material has no more than three amino acid residues; and said complex includes at least one metallic ion selected from the group consisting of: Zn++, Ca++, Mg++, Mn++, and Fe++.

10. A composition of claim 2, characterized further by the additional limitations that said aqueous-phosphate solution is approximately 0.02 M phosphate, the pH thereof is adjusted to approximately 5, and the acetonitrile input concentration of said gradient includes at least the range from approximately slightly above 0% to approximately 15%.

11. A composition of claim 10 characterized further by the additional additional limitations that said material:
(a) is primarily O.S. elutable in the portions of said gradient where the effluent has an acetonitrile concentration between approximately 0.2% and approximately 1.5%; is substantially not O.S. elutable in the portions of said gradient where the effluent has an acetonitrile concentration below approximately 0.2%; is largely free from material O.S. elutable in the portions of said gradient where the effluent has an acetonitrile concentration below approximately 0.2%; and is largely free from material O.S. elutable primarily, when said gradient is of monotonically increasing acetonitrile concentration, in the portions of said gradient where the effluent has an acetonitrile concentration greater than approximately 1.5%;
(b) is also O.S. elutable with an ACTF 0.1% gradient; is primarily O.S. elutable in the portions of said ACTF gradient where the effluent has an acetonitrile concentration between approximately 11.8% and approximately 14.8%; is substantially not O.S. elutable in the portions of said gradient where the effluent has an acetonitrile concentration below approximately 11.8%; is largely free from material O.S. elutable in the portions of said gradient where the effluent has an acetonitrile concentration below approximately 11.8%; and is largely free from material O.S. elutable primarily, when said gradient is of monotonically increasing acetonitrile concentration, in the portions of said gradient where the effluent has an acetonitrile concentration greater than approximately 14.8%; and
(c) is also O.S. elutable with an ethanol-in-water gradient including at least the range of input ethanol concentration from below approximately 0.1% to above approximately 10%; is primarily O.S. elutable in the portions of said ethanol-in-water gradient where the effluent has an ethanol concentration between approximately 0.1% and approximately 0.4%; is substantially not O.S. elutable in the portions of said gradient where the effluent has an ethanol concentration below approximately 0.1%; is largely free from material O.S. elutable in the portions of said gradient where the effluent has an ethanol concentration below approximately 0.4%; and is largely free from material O.S. elutable primarily, when said gradient is of monotonically increasing ethanol concentration, in the portions of said gradient where the effluent has an ethanol concentration greater than approximately 0.4% --
said further limited material of this claim being hereinafter designated "Beta-1.1."

12. A composition of claim 11 further characterized by the additional limitations that said material:
(a) is also O.S. elutable in an ACTF 0.05% gradient;
(b) elutes from said gradient in approximately the middle of the input range from 10% to 20% acetonitrile concentration;
(c) consists of dipeptide material wherein Tyr-Gly is the only amino acid residue sequence; and
(d) has a UVAP with reference to said gradient and 210 nm of being approximately the fourth sharp, narrow, distinct peak --
said material being hereinafter designated "Beta-1.11."

13. A composition of claim 11 further characterized by the additional limitations that said material:
(a) is O.S. elutable in an ACTF 0.05% gradient;
(b) elutes from said gradient in approximately the first third of the input range from 10% to 20% acetonitrile concentration;
(c) consists of tripeptide material wherein Tyr-Gly-Gly is the only amino acid residue sequence; and
(d) has a UVAP with reference to said gradient and 210 nm of being approximately the third sharp, narrow, distinct peak --
said material being hereinafter designated "Beta-1.12."

14. A composition of claim 1 packaged in a pharmaceutically acceptable vehicle and in an effective dosage amount, whereby a pharmaceutical composition is provided.

15. The pharmaceutical composition of claim 14 wherein the composition of claim 1 includes at least one small peptide amplifier.

16. The pharmaceutical composition of claim 14 wherein there is added an effective amount of an antigen or pathogen, thereby providing a vaccine composition.

17. The pharmaceutical composition of claim 14 wherein there is added an effective amount of a diagnostic antigen or pathogen, thereby providing a composition for diagnosing prior exposure to said antigen or pathogen.

18. Use of the composition according to claim 1 in an effective dosage amount and in a pharmaceutically acceptable carrier for amplifying the immune response of a person to the introduction of antigen of which said person has previously been exposed or is concurrently exposed.

19. The use of claim 18 wherein said polypeptide material of the composition includes at least one small peptide amplifier.

20. The use of claim 18 wherein said person has an immune deficiency disease or hypoimmune condition.

21. The use of claim 20 where said disease is AIDS or ARC.

22. The use of claim 20 wherein said disease is associated with a defect in T-helper cell function.

23. The use of claim 20 wherein the composition is applied in combination with a transfusion containing T-helper cells.

24. The use of claim 18 wherein said dosage is applied via a skin pad at a skin site on said person.

25. Use of a pharmaceutical composition containing at least one molecular species wherein a Tyr-Gly aminoacid residue sequence occurs in an effective dosage amount of a cell-mediated immune system function of a human or mammalian subject.

26. The use of claim 25 wherein the Tyr residue is at the N-terminal end of an amino acid residue sequence.

27. The use of claim 26 wherein said Tyr residue is N-methylated.

28. The use of claim 25 wherein said molecular species contains a D-aminoacid residue inserted at a location other than at the N-terminal end of the amino acid residue sequence, and where said location is one where there is a peptide bond that, in the absence of said D-aminoacid residue, is susceptible to cleavage by action of an enzyme present in the body of said subject.

29. The use of claim 25 wherein an amino acid residue in said molecular species is methylated or amidified at a location where there is a peptide bond that, in the absence of said methylation or amidification, is susceptible to cleavage by action of an enzyme present in the body of said subject.

30. The use of claim 25 wherein said polypeptide material contains at least one of the following materials: Tyr-Gly material and Tyr-Gly-Gly material.

31. The use of claim 25 wherein said molecular species metabolizes in vivo to a Tyr-Gly material or a Tyr-Gly-Gly material.

32. A pharmaceutical composition comprising a pharmaceutically acceptable carrier and an effective dosage amount of Tyr-Gly, Tyr-Gly-Gly, or a pharmaceutically acceptable derivative thereof, free of pharmaceutically unacceptable constituents.

33. A pharmaceutical composition comprising a pharmaceutically acceptable carrier and an effective dosage amount of a pharmaceutically acceptable material that metabolizes in vivo to a Tyr-Gly material or a Tyr-Gly-Gly material.

34. In a process for purifying Beta material wherein a HPLC column containing a starter Beta material preparation is eluted with a gradient of acetonitrile in dilute aqueous phosphate solution, thereby producing a partially purified amplifier material, wherein said partially purified amplifier material is subjected to further HPLC with an ethanol-in-water gradient, thereby producing a further purified amplifier material substantially free of phenylalanine previously associated therewith, the improvement comprising subjecting said further purified material to the following further HPLC steps:
(1) applying said further purified material to a reverse-phase HPLC column, packed with octadecylsilane;
(2) eluting said column with an acetonitrile-in-aqueous-trifluoroacetic-acid gradient that includes the input range from below approximately 10% acetonitrile concentration to above approximately 20% and wherein said trifluoroacetic acid concentration is approximately 0.05% (approximate PH 2.5), thereby producing a plurality of further effluent-fractions;
(3) monitoring the UV absorbance of the effluent from said column in the zone of approximately 210 nm; and
(4) selecting predetermined further effluent-fractions and collecting them.

35. The process of claim 34 wherein said further effluent fractions that are selected and collected are ones associated with a sharp, narrow, distinct peak that is approximately the fourth peak.

36. The process of claim 34 wherein said further effluent fractions that are selected and collected are ones associated with a sharp, narrow, distinct peak that is approximately the third peak.

37. The process of claim 34 wherein said column has been calibrated for retention time by passing therethrough a material known to be Tyr-Gly-Gly material; said retention time of said material has been thus determined; and said retention time is subsequently utilized, when using said column, to provide an indication that said predetermined further effluent fractions are about to elute.

38. The process of claim 34 wherein labelled Tyr-Gly-Gly material is passed through the column to provide an indication that said predetermined further effluent fractions are about to elute.

39. A diagnostic reagent article for testing persons for exposure to tuberculosis bacteria, comprising a vial containing an effective dosage amount of tuberculin in a pharmaceutically acceptable liquid carrier, and an effective dosage amount of a pharmaceutical composition containing Tyr-Gly, Tyr-Gly-Gly, or a derivative thereof.

## Patentansprüche

1. Stoffzusammensetzung, die gekennzeichnet ist durch die Eigenschaften, daß sie:
(a) die Geschwindigkeit, Größe oder Dauer einer Antwort des Immunsystems eines menschlichen Körpers erhöht, wobei diese Antwort:
(1) auf die Einführung mindestens eines Antigens in den Körper erfolgt, dem dieser Körper vorher ausgesetzt war oder momentan ausgesetzt ist,
(2) speziell einer Funktion des menschlichen Immunsystems zuzuschreiben ist und
(3) nach der Einführung des Antigens auftritt;
(b) im wesentlichen frei von anderem, davon verschiedenem Material ist, das eine Auswirkung auf die Immunsystem-Antwort hat, die der Auswirkung der Stoffzusammensetzung auf diese Antwort entgegengesetzt ist;
(c) im wesentlichen frei von Substanzen ist, die eine Antigen-spezifische Immunsystem-Antwort auf ein Antigen übertragen, dem dieser Körper bisher nicht ausgesetzt war und momentan nicht ausgesetzt ist;
(d) im wesentlichen aus Polypeptidmaterialien besteht, die ein mittleres Molekulargewicht von mehr als ca. 200 und weniger als ca. 1500 haben;
(e) mit einem Gradienten von Acetonitril in wäßriger Phosphatlösung O.S.-eluierbar ist; und
(f) im wesentlichen frei von pharmazeutisch inakzeptablen Bestandteilen ist.

2. Zusammensetzung nach Anspruch 1, wobei das Polypeptidmaterial Moleküle hat, die nicht mehr als sechs Peptidgruppen enthalten.

3. Zusammensetzung nach Anspruch 2, wobei das Polypeptidmaterial ein Dipeptid ist, das Tyr-Gly als die einzige Aminosäurerest-Sequenz enthält.

4. Zusammensetzung nach Anspruch 2, wobei das Polypeptidmaterial ein Tripeptid ist, das Tyr-Gly-Gly als die einzige Aminosäurerest-Sequenz enthält.

5. Zusammensetzung nach Anspruch 3 oder 4, wobei der Tyr-Rest N-methyliert ist.

6. Zusammensetzung nach Anspruch 3 oder 4, wobei die Carboxylgruppe am C-terminalen Ende der Aminosäurerest-Sequenz amidiert oder verestert ist.

7. Zusammensetzung nach Anspruch 2, die in dem Polypeptidmaterial einen D-Aminosäurerest in einer anderen Position als am N-terminalen Ende der Aminosäurerest-Sequenz enthält.

8. Zusammensetzung nach Anspruch 2, an die ein Hemmstoff gebunden ist.

9. Zusammensetzung nach Anspruch 2, wobei mindestens zwei Polypeptidmaterialien einen Komplex bilden; jedes Material nicht mehr als drei Aminosäurereste hat; und der Komplex mindestens ein Metallion aufweist, das aus der Gruppe ausgewählt ist, die aus Zn++, Ca++, Mg++, Mn++ und Fe++ besteht.

10. Zusammensetzung nach Anspruch 2, ferner gekennzeichnet durch die zusätzlichen Einschränkungen, daß die wäßrige Phosphatlösung angenähert 0,02 M Phosphat ist, daß ihr pH auf angenähert 5 eingestellt ist und daß die Acetonitril-Eingangskonzentration des Gradienten mindestens den Bereich von angenähert geringfügig über 0 % bis angenähert 15 % umfaßt.

11. Zusammensetzung nach Anspruch 10, ferner gekennzeichnet durch die weiteren zusätzlichen Einschränkungen, daß das Material:
(a) primär O.S.-eluierbar ist in den Bereichen des Gradienten, in denen der Abfluß eine Acetonitrilkonzentration zwischen angenähert 0,2 % und angenähert 1,5 % hat; im wesentlichen nicht O.S.-eluierbar ist in den Bereichen des Gradienten, in denen der Abfluß eine Acetonitrilkonzentration unter angenähert 0,2 % hat; weitgehend frei ist von Material, das O.S.-eluierbar ist in den Bereichen des Gradienten, in denen der Abfluß eine Acetonitrilkonzentration unter angenähert 0,2 % hat; und weitgehend frei von Material ist, das, wenn der Gradient monotonisch zunehmende Acetonitrilkonzentration hat, primär in den Bereichen des Gradienten O.S.-eluierbar ist, in denen der Abfluß eine Acetonitrilkonzentration von mehr als angenähert 1,5 % hat;
(b) außerdem mit einem 0,1 % ACTF-Gradienten O.S.-eluierbar ist; primär O.S.-eluierbar ist in den Bereichen des ACTF-Gradienten, in denen der Abfluß eine Acetonitrilkonzentration zwischen angenähert 11,8 % und angenähert 14,8 % hat; im wesentlichen nicht O.S.-eluierbar ist in den Bereichen des Gradienten, in denen der Abfluß eine Acetonitrilkonzentration unter angenähert 11,8 % hat; weitgehend frei von Material ist, das in den Bereichen des Gradienten O.S.-eluierbar ist, in denen der Abfluß eine Acetonitrilkonzentration unter angenähert 11,8 % hat; und weitgehend frei von Material ist, das, wenn der Gradient monotonisch zunehmende Acetonitrilkonzentration hat, primär in den Bereichen des Gradienten O.S.-eluierbar ist, in denen der Abfluß eine Acetonitrilkonzentration von mehr als angenähert 14,8 % hat; und
(c) außerdem mit einem Ethanol-in-Wasser-Gradienten O.S.-eluierbar ist, der mindestens den Bereich einer Ethanol-Eingangskonzentration von unter angenähert 0,1 % bis über angenähert 10 % umfaßt; primär in den Bereichen des Ethanol-in-Wasser-Gradienten O.S.-eluierbar ist, in denen der Abfluß eine Ethanolkonzentration zwischen angenähert 0,1 % und angenähert 0,4 % hat; im wesentlichen nicht O.S.-eluierbar ist in den Bereichen des Gradienten, in denen der Abfluß eine Ethanolkonzentration unter angenähert 0,1 % hat; weitgehend frei von Material ist, das in den Bereichen des Gradienten O.S.-eluierbar ist, in denen der Abfluß eine Ethanolkonzentration unter angenähert 0,4 % hat; und weitgehend frei von Material ist, das, wenn der Gradient monotonisch zunehmende Ethanolkonzentration hat, primär in den Bereichen des Gradienten O.S.-eluierbar ist, in denen der Abfluß eine Ethanolkonzentration von mehr als angenähert 0,4 % hat;
wobei dieses weiter eingeschränkte Material des vorliegenden Anspruchs nachstehend mit "Beta-1.1." bezeichnet ist.

12. Zusammensetzung nach Anspruch 11, ferner gekennzeichnet durch die zusätzlichen Einschränkungen, daß das Material:
(a) außerdem in einem 0,05 % ACTF-Gradienten O.S.-eluierbar ist;
(b) aus dem Gradienten angenähert in der Mitte des Eingangsbereichs von 10 % bis 20 % Acetonitrilkonzentration eluiert;
(c) aus Dipeptidmaterial besteht, wobei Tyr-Gly die einzige Aminosäurerest-Sequenz ist; und
(d) ein UVAP (UV-Absorptionsprofil) in bezug auf den Gradienten und 210 nm hat, wobei es ungefähr der vierte scharfe, schmale, deutliche Peak ist,
wobei dieses Material nachstehend mit "Beta-1.11." bezeichnet ist.

13. Zusammensetzung nach Anspruch 11, ferner gekennzeichnet durch die zusätzlichen Einschränkungen, daß das Material:
(a) in einem 0,05 % ACTF-Gradienten O.S.-eluierbar ist;
(b) aus dem Gradienten in angenähert dem ersten Drittel des Eingangsbereichs einer Acetonitrilkonzentration von 10 % bis 20 % eluiert;
(c) aus Tripeptidmaterial besteht, in dem Tyr-Gly-Gly die einzige Aminosäurerest-Sequenz ist; und
(d) ein UVAP in bezug auf den Gradienten und 210 nm hat, wobei es ungefähr der dritte scharfe, schmale, deutliche Peak ist,
wobei das Material nachstehend mit "Beta-1.12." bezeichnet ist.

14. Zusammensetzung nach Anspruch 1, die in einem pharmazeutisch akzeptablen Träger und in einer wirksamen Dosismenge abgepackt ist, wodurch eine pharmazeutische Zusammensetzung erhalten wird.

15. Pharmazeutische Zusammensetzung nach Anspruch 14, wobei die Zusammensetzung von Anspruch 1 mindestens einen kleinen Peptid-Amplifikator aufweist.

16. Pharmazeutische Zusammensetzung nach Anspruch 14, wobei eine wirksame Menge eines Antigens oder Pathogens zugegeben ist, um dadurch eine Impfstoffzusammensetzung zu erhalten.

17. Pharmazeutische Zusammensetzung nach Anspruch 14, wobei eine wirksame Menge eines diagnostischen Antigens oder Pathogens zugegeben ist, um dadurch eine Zusammensetzung zur diagnostischen Bestimmung eines früheren Ausgesetztseins gegenüber diesem Antigen oder Pathogen zu erhalten.

18. Verwendung der Zusammensetzung nach Anspruch 1 in einer wirksamen Dosismenge und in einem pharmazeutisch akzeptablen Träger zur Amplifizierung der Immunantwort einer Person auf die Einführung eines Antigens, dem diese Person früher ausgesetzt war oder momentan ausgesetzt ist.

19. Verwendung nach Anspruch 18, wobei das Polypeptidmaterial der Zusammensetzung mindestens einen kleinen Peptid-Amplifikator aufweist.

20. Verwendung nach Anspruch 18, wobei die Person einen Immunmangelerkrankungs- oder Immunschwächezustand hat.

21. Verwendung nach Anspruch 20, wobei die Erkrankung AIDS oder ARC ist.

22. Verwendung nach Anspruch 20, wobei die Erkrankung mit einem Defekt der T-Helferzellenfunktion verbunden ist.

23. Verwendung nach Anspruch 20, wobei die Zusammensetzung in Kombination mit einer T-Helferzellen enthaltenden Transfusion angewandt wird.

24. Verwendung nach Anspruch 18, wobei die Dosis über ein Hautpflaster auf eine Hautstelle an der genannten Person aufgebracht wird.

25. Verwendung einer pharmazeutischen Zusammensetzung, die mindestens eine Molekülspezies enthält, wobei eine Tyr-Gly-Aminosäurerest-Sequenz in einer wirksamen Dosismenge einer zellvermittelten Immunsystemfunktion eines Human- oder Säugetier-Subjekts auftritt.

26. Verwendung nach Anspruch 25, wobei der Tyr-Rest an dem N-terminalen Ende einer Aminosäurerest-Sequenz vorhanden ist.

27. Verwendung nach Anspruch 26, wobei der Tyr-Rest N-methyliert ist.

28. Verwendung nach Anspruch 25, wobei die Molekülspezies einen D-Aminosäurerest enthält, der an einer anderen Stelle als dem N-terminalen Ende der Aminosäurerest-Sequenz eingefügt ist, und wobei diese Stelle eine ist, an der sich eine Peptidbindung befindet, die in Abwesenheit des D-Aminosäurerests für Spaltung durch die Wirkung eines Enzyms, das in dem Körper des Subjekts vorhanden ist, empfindlich ist.

29. Verwendung nach Anspruch 25, wobei ein Aminosäurerest in der Molekülspezies an einer Stelle methyliert oder amidiert ist, an der sich eine Peptidbindung befindet, die in Abwesenheit dieser Methylierung oder Amidierung für Spaltung durch die Wirkung eines Enzyms, das in dem Körper des Subjekts vorhanden ist, empfindlich ist.

30. Verwendung nach Anspruch 24, wobei das Polypeptidmaterial mindestens eines der folgenden Materialien enthält: Tyr-Gly-Material und Tyr-Gly-Gly-Material.

31. Verwendung nach Anspruch 25, wobei die Molekülspezies in vivo zu einem Tyr-Gly-Material oder einem Tyr-Gly-Gly-Material umgesetzt wird.

32. Pharmazeutische Zusammensetzung, die einen pharmazeutisch akzeptablen Träger und eine wirksame Dosismenge von Tyr-Gly, Tyr-Gly-Gly oder eines pharmazeutisch akzeptablen Derivats davon aufweist, wobei die Zusammensetzung frei von pharmmazeutisch nichtakzeptablen Bestandteilen ist.

33. Pharmazeutische Zusammensetzung, die einen pharmazeutisch akzeptablen Träger und eine wirksame Dosismenge eines pharmazeutisch akzeptablen Materials aufweist, das in vivo zu einem Tyr-Gly-Material oder einem Tyr-Gly-Gly-Material umgesetzt wird.

34. Verfahren zum Reinigen von Beta-Material, wobei eine HPLC-Säule, die ein Starter-Betamaterial-Präparat enthält, mit einem Gradienten von Acetonitril in verdünnter wäßriger Phosphatlösung eluiert wird, um dadurch ein teilgereinigtes Amplifikatormaterial zu produzieren, wobei das teilgereinigte Amplifikatormaterial weiterer HPLC mit einem Ethanol-in-Wasser-Gradienten unterworfen wird, um dadurch ein weiter gereinigtes Amplifikatormaterial zu erzeugen, das im wesentlichen frei von Phenylalanin ist, das vorher damit assoziiert war, wobei die Verbesserung aufweist: Unterwerfen des weiter gereinigten Materials den folgenden weiteren HPLC-Schritten:
(1) Aufgeben des weiter gereinigten Materials auf eine Umkehrphasen-HPLC-Säule, die mit Octadecylsilan gepackt ist;
(2) Eluieren dieser Säule mit einem Gradienten von Acetonitril in wäßriger Trifluoressigsäure, der den Eingangsbereich von unter angenähert 10 % Acetonitrilkonzentration bis über angenähert 20 % umfaßt, und wobei die Trifluoressigsäurekonzentration angenähert 0,05 % (ungefährer pH 2,5) ist, um dadurch eine Vielzahl von weiteren Abfluß-Fraktionen zu produzieren;
(3) Überwachen des UV-Absorptionsvermögens des Abflusses aus der Säule in der Zone von angenähert 210 nm; und
(4) Wählen vorbestimmter weiterer Abfluß-Fraktionen und Sammeln derselben.

35. Verfahren nach Anspruch 34, wobei die weiteren Abfluß-Fraktionen, die gewählt und gesammelt werden, solche sind, die einem scharfen, schmalen, deutlichen Peak zugeordnet sind, der ungefähr der vierte Peak ist.

36. Verfahren nach Anspruch 34, wobei die weiteren Abfluß-Fraktionen, die gewählt und gesammelt werden, solche sind, die einem scharfen, schmalen, deutlichen Peak zugeordnet sind, der ungefähr der dritte Peak ist.

37. Verfahren nach Anspruch 34, wobei die Säule Retentionszeit-kalibriert worden ist durch Hindurchleiten eines Materials, von dem bekannt ist, daß es Tyr-Gly-Gly-Material ist; wobei die Retentionszeit des Materials auf diese Weise bestimmt worden ist; und wobei die Retentionszeit anschließend im Gebrauch der Säule genutzt wird, um eine Anzeige zu liefern, daß die Elution der vorbestimmten weiteren Abfluß-Fraktionen unmittelbar bevorsteht.

38. Verfahren nach Anspruch 34, wobei markiertes Tyr-Gly-Gly-Material durch die Säule geschickt wird, um eine Anzeige dafür zu liefern, daß die Elution der vorbestimmten weiteren Abfluß-Fraktionen unmittelbar bevorsteht.

39. Diagnostischer Reagensgegenstand, um Personen daraufhin zu testen, ob sie Tuberkulosebakterien ausgesetzt sind, wobei der Gegenstand aufweist: eine Ampulle, die folgendes enthält: eine wirksame Dosismenge Tuberculin in einem pharmazeutisch akzeptablen flüssigen Träger und eine wirksame Dosismenge einer pharmazeutischen Zusammensetzung, die Tyr-Gly, Tyr-Gly-Gly oder ein Derivat davon enthält.

## Revendications

1. Composition de matière, caractérisée en ce que :
(a) elle augmente la vitesse, l'importance ou la durée d'une réponse du système immunitaire d'un corps humain, lorsque cette réponse :
(1) est à l'introduction dans ce corps d'au moins un antigène auquel le corps a été précédemment ou est simultanément exposé,
(2) peut être spécifiquement attribuée à une fonction du système immunitaire humain, et
(3) a lieu après l'introduction de cet antigène;
(b) elle est pratiquement dépourvue d'un autre matériau différent qui a un effet sur cette réponse du système immunitaire opposé à l'effet de cette composition de matière sur cette réponse;
(c) elle est pratiquement dépourvue de substance qui transfère une réponse du système immunitaire spécifique de l'antigène à un antigène auquel ce corps n'a pas été précédemment exposé ou n'est pas simultanément exposé;
(d) elle consiste essentiellement en matériaux polypeptides ayant un poids moléculaire moyen supérieur à environ 200 et inférieur à environ 1500;
(e) est éluable de O.S. avec un gradient d'acétonitrile dans une solution aqueuse de phosphate; et
(f) elle est pratiquement dépourvue de constituants pharmaceutiquement inacceptables.

2. Composition selon la revendication 1, dans lequelle le matériau polypeptide a des molécules ne contenant pas plus de six groupes peptides.

3. Composition selon la revendication 2, dans laquelle le matériau polypeptide est un dipeptide contenant Tyr-Gly comme seule séquence de résidus d'acides aminés.

4. Composition selon la revendication 2, dans laquelle le matériau polypeptide est un tripeptide contenant Tyr-Gly-Gly comme seule séquence de résidus d'acides aminés.

5. Composition selon la revendication 3 ou la revendication 4, dans laquelle le résidu de Tyr est méthylé en N.

6. Composition selon la revendication 3 ou la revendication 4, dans laquelle le groupe carboxyle situé à l'extrémité C-terminale de la séquence de résidus d'acides aminés est amidifié ou estérifié.

7. Composition selon la revendication 2, contenant dans le matériau polypeptide un résidu de D-aminoacide dans une position autre qu'à l'extrémité N-terminale de la séquence de résidus d'acides aminés.

8. Composition selon la revendication 2, à laquelle est lié un inhibiteur.

9. Composition selon la revendication 2, dans laquelle au moins deux matériaux polypeptides forment un complexe, chaque matériau n'a pas plus de trois résidus d'acides aminés; et le complexe comprend au moins un ion métallique sélectionné dans le groupe comprenant Zn++, Ca++, Mg++, Mn++ et Fe++.

10. Composition selon la revendication 2, caractérisée en outre par les limitations additionnelles que la solution aqueuse de phosphate est une solution de phosphate environ 0,02 M, que son pH est ajusté à approximativement 5 et que la concentration d'acétonitrile à l'entrée de ce gradient comprend au moins la plage allant d'environ légèrement au-dessus de 0% à environ 15%.

11. Composition selon la revendication 10, caractérisée en outre par les limitations additionnelles que ce matériau :
(a) est principalement éluable de O.S. dans les portions de ce gradient où l'effluent a une concentration en acétonitrile comprise entre environ 0,2% et environ 1,5%, est pratiquement non-éluable de O.S. dans les portions de ce gradient où l'effluent a une concentration en acétonitrile inférieure à environ 0,2%; est largement dépourvu de matériaux éluables de O.S. dans les portions de ce gradient où l'effluent a une concentration d'acétonitrile inférieure à environ 0,2%; et est largement dépourvu de matériaux principalement éluables de O.S., lorsque la concentration d'acétonitrile de ce gradient croît uniformément, dans les portions de ce gradient où l'effluent a une concentration d'acétonitrile supérieure à environ 1,5%;
(b) est également éluable de O.S. avec un gradient ACTF 0,1%; est principalement éluable de O.S. dans la portion de ce gradient ACTF où l'effluent a une concentration d'acétonitrile comprise entre environ 11,8% et environ 14,8%, est pratiquement non-éluable de O.S. dans les portions de ce gradient où l'effluent a une concentration d'acétonitrile inférieure à environ 11,8%; est largement dépourvu de matériaux éluables de O.S. dans les portions de ce gradient où l'effluent a une concentration d'acétonitrile inférieure à environ 11,8%; et est largement dépourvu de matériaux principalement éluables de O.S., lorsque la concentration d'acétonitrile de ce gradient croît uniformément, dans les portions de ce gradient où l'effluent a une concentration d'acétonitrile supérieure à environ 14,8%; et
(c) est également éluable de O.S. avec un gradient d'éthanol dans l'eau comprenant au moins la plage de concentrations d'éthanol à l'entrée allant de moins d'environ 0,1% à plus d'environ 10%; est principalement éluable de O.S. dans les portions de ce gradient d'éthanol dans l'eau où l'effluent a une concentration d'éthanol comprise entre environ 0,1% et environ 0,4%, est pratiquement non-éluable de O.S. dans les portions de ce gradient où l'effluent a une concentration d'éthanol inférieure à environ 0,1%; est largement dépourvu de matériaux éluables de O.S. dans les portions de ce gradient où l'effluent a une concentration d'éthanol inférieure à environ 0,4%; et est largement dépourvu de matériaux principalement éluables de O.S., lorsque la concentration d'éthanol de ce gradient croît régulièrement, dans les portions de ce gradient où l'effluent a une concentration d'éthanol supérieure à environ 0,4%,
ce matériau ainsi limité de cette revendication étant ci-après désigné "Bêta-1.1".

12. Composition selon la revendication 11, caractérisée en outre par la limitation additionnelle que ce matériau :
(a) est également éluable de O.S. dans un gradient ACTF 0,05%;
(b) elue de ce gradient dans approximativement le milieu de la plage de concentrations d'acétonitrile à l'entrée comprise entre 10 et 20%;
(c) consiste en le matériau dipeptide dans lequel Tyr-Gly est la seule séquence de résidus d'acides aminés; et
(d) a un PAUV par référence à ce gradient et 210 nm d'être environ le quatrième pic distinct, étroit et aigu,
ce matériau étant ci-après désigné "Bêta-1.11".

13. Composition selon la revendication 11, caractérisée en outre par les limitations additionnelles que ce matériau :
(a) est éluable de O.S. dans un gradient ACTF 0,05%;
(b) élue de ce gradient dans approximativement le premier tiers de la plage de concentrations d'acétonitrile à l'entrée comprise entre 10 et 20%;
(c) est constitué d'un matériau tripeptide, dans lequel Tyr-Gly-Gly est la seule séquence de résidus d'acides aminés; et
(d) a un PAUV par référence à ce gradient et 210 nm d'être environ le troisième pic distinct, étroit et aigu, ce matériau étant ci-après désigné "Bêta-1.12".

14. Composition selon la revendication 1, emballée dans un véhicule pharmaceutiquement acceptable et en une quantité de dosage efficace, d'où il résulte qu'on obtient une composition pharmaceutique.

15. Composition pharmaceutique selon la revendication 14, dans laquelle la composition de la revendication 1 comprend au moins un petit peptide amplificateur.

16. Composition pharmaceutique selon la revendication 14, dans laquelle il est ajouté une quantité efficace d'un antigène ou d'un pathogène, procurant ainsi une composition de vaccin.

17. Composition pharmaceutique selon la revendication 14, dans laquelle il est ajouté une quantité efficace d'un antigène ou pathogène diagnostique, procurant ainsi une composition pour effectuer un diagnostic avant exposition à cet antigène ou ce pathogène.

18. Utilisation de la composition selon la revendication 1 en une quantité de dosage efficace et dans un support pharmaceutiquement acceptable pour amplifier la réponse immunitaire d'une personne à l'introduction d'un antigène auquel cette personne a été précédemment exposée ou est simultanément exposée.

19. Utilisation selon la revendication 18, dans laquelle le matériau polypeptide de la composition comprend au moins un petit peptide amplificateur.

20. Utilisation selon la revendication 18, dans laquelle cette personne a une maladie de déficience immunitaire ou un état hypo-immun.

21. Utilisation selon la revendication 20, dans laquelle cette maladie est le SIDA ou le para-SIDA.

22. Utilisation selon la revendication 20, dans laquelle la maladie est associée à un défaut de la fonction des cellules T auxiliaires.

23. Utilisation selon la revendication 20, dans laquelle la composition est appliquée en combinaison avec une transfusion contenant des cellules T auxiliaires.

24. Utilisation selon la revendication 18, dans laquelle ce dosage est appliqué par l'intermédiaire d'une pastille dermique sur un site de la peau de cette personne.

25. Utilisation d'une composition pharmaceutique contenant au moins une espèce moléculaire dans laquelle une séquence de résidus d'acides aminés Tyr-Gly existe en une quantité de dosage efficace d'une fonction du système immunitaire à médiation cellulaire d'une personne ou d'un mammifère.

26. Utilisation selon la revendication 25, dans laquelle le résidu Tyr est situé à l'extrémité N-terminale d'une séquence de résidus d'acides aminés.

27. Utilisation selon la revendication 26, dans laquelle le résidu Tyr est méthylé en N.

28. Utilisation selon la revendication 25, dans laquelle l'espèce moléculaire contient un résidu de D-aminoacide inséré à un emplacement autre qu'à l'extrémité N-terminale de la séquence de résidus d'acides aminés et dans laquelle cet emplacement est un emplacement où il y a une liaison peptide qui, en l'absence de ce résidu D-aminoacide, est susceptible de clivage par action d'une enzyme présente dans le corps du sujet.

29. Utilisation selon la revendication 25, dans laquelle un résidu d'acide aminé dans cette espèce moléculaire est méthylé ou amidifié à un emplacement où il y a une liaison peptide qui, en l'absence de cette méthylation ou amidification, est susceptible de clivage par l'action d'une enzyme présente dans le corps du sujet.

30. Utilisation selon la revendication 25, dans laquelle le matériau polypeptide contient au moins l'un des matériaux suivants : matériau Tyr-Gly et matériau Tyr-Gly-Gly.

31. Utilisation selon la revendication 25, dans laquelle l'espèce moléculaire se métabolise in vivo en un matériau Tyr-Gly ou un matériau Tyr-Gly-Gly.

32. Composition pharmaceutique comprenant un support pharmaceutiquement acceptable et un dosage efficace de Tyr-Gly, Tyr-Gly-Gly, ou un dérivé pharmaceutiquement acceptable de ces substances, dépourvu de constituants pharmaceutiquement inacceptables.

33. Composition pharmaceutique comprenant un support pharmaceutiquement acceptable et un dosage efficace d'un matériau pharmaceutiquement acceptable qui se métabolise in vivo en un matériau Tyr-Gly ou un matériau Tyr-Gly-Gly.

34. Dans un procédé pour purifier un matériau Bêta, dans lequel une colonne de CLHP contenant une préparation de matériau Bêta de départ est éluée avec un gradient d'acétonitrile dans une solution aqueuse diluée de phosphate, produisant ainsi un matériau amplificateur partiellement purifié, dans lequel ce matériau amplificateur partiellement purifié est soumis à une autre CLHP avec un gradient d'éthanol dans l'eau, produisant ainsi un matériau amplificateur davantage purifié pratiquement dépourvu de la phénylalanine qui lui était précédemment associée, le perfectionnement consistant à soumettre ce matériau davantage purifié aux étapes d'une CLHP ultérieure suivantes :
(1) appliquer ce matériau davantage purifié sur une colonne de CLHP en phase inverse, remplie d'octadécylsilane;
(2) éluer cette colonne avec un gradient d'acétonitrile dans une solution aqueuse d'acide trifluoroacétique, qui comprend la plage de concentrations d'acétonitrile à l'entrée allant approximativement d'en dessous de 10% jusqu'au-dessus de 20% et dans lequel la concentration d'acide trifluoroacétique est environ 0,05% (pH approximatif 2,5), produisant ainsi une multiplicité d'autres fractions d'effluents;
(3) contrôler l'absorbance des U.V. de l'effluent sortant de cette colonne dans la zone d'environ 210 nm; et
(4) sélectionner d'autres fractions d'effluents prédéterminées et les recueillir.

35. Procédé selon la revendication 34, dans lequel ces autres fractions d'effluents qui sont sélectionnées et recueillies sont des fractions associées à un pic distinct, étroit et aigu qui est approximativement le quatrième pic.

36. Procédé selon la revendication 34, dans lequel ces autres fractions d'effluents qui sont sélectionnées et recueillies sont des fractions associées à un pic distinct étroit et aigu qui est approximativement le troisième pic.

37. Procédé selon la revendication 34, dans lequel la colonne a été étalonnée en ce qui concerne les temps de rétention en faisant passer à travers elle un matériau connu pour être le matériau Tyr-Gly-Gly, le temps de rétention de ce matériau a été ainsi déterminé et ce temps de rétention est ensuite utilisé, lorsqu'on utilise cette colonne, pour procurer une indication que ces autres fractions prédéterminées d'effluents sont sur le point d'éluer.

38. Procédé selon la revendication 34, dans lequel un matériau Tyr-Gly-Gly marqué passe à travers la colonne pour procurer une indication que ces autres fractions d'effluents prédéterminées sont sur le point d'éluer.

39. Réactif diagnostique pour tester des personnes pour exposition au bacille de la tuberculose, comprenant une fiole contenant un dosage efficace de tuberculine dans un support liquide pharmaceutiquement acceptable, et un dosage efficace d'une composition pharmaceutique contenant Tyr-Gly, Tyr-Gly-Gly ou un dérivé de ces substances.
